# EUROPEAN PATENT APPLICATION

(11) **EP 2 272 982 A1**
(43) Date of publication of application: **12.01.2011**
(21) Application number: 10151937.9
(22) Date of filing: 20.08.2007
(51) Int. Cl.: C12Q 1/68

(54) **Method for treatment of prostate cancer and diagnosing of patients benefiting from the same**

(30) Priority: 23.08.2006 US 839416 P; 23.08.2006 FI 20060751
(62) Divisional of application: 07803694.4
(71) Applicant: VALTION TEKNILLINEN TUTKIMUSKESKUS, 02150 Espoo (FI)
(72) Inventor: Iljin, Kristiina, 20900 Turku (FI); Nees, Matthias, 20610 Turku (FI); Kallioniemi, Olli, 21610 Kirjala (FI); Björkman, Mari, 20880 Turku (FI)
(74) Representative: Suominen, Kaisa Liisa

(57) **Abstract**

The invention relates to a method for treating ERG-positive prostate cancer patients with an agent counteracting one or more ERG-associated genes and/or manipulating of one or more ERG-related pathways, optionally in combination with an androgen deprivation therapy wherein at least one ERG-associated gene HDAC1 is used. Furthermore, the invention concerns methods for screening prostate cancer patients which may benefit from said treatment, assessing the efficacy of a therapy for treating prostate cancer in a patient, assessing progression of prostate cancer in a patient, selecting an agent to be tested for usefulness in the treatment of prostate cancer, and for assessing prostate carcinogenic potential of an agent.

SEQUENCE LISTING

<110> Valtion teknillinen tutkimuskeskus
Iljin, Kristiina
Nees, Matthias
Kallioniemi, Olli

<120> Method for Treatment of Prostate Cancer and Screening of Patients
Benefiting from Said Method

<130> ERG HDAC1/VP1003

<160> 14

<170> PatentIn version 3.3

<210> 1
<211> 22
<212> DNA
<213> Artificial

<220>
<223> oligonucleotide primer

<400> 1
aagtatcacc agagggtgct gt 22

<210> 2
<211> 22
<212> DNA
<213> Artificial

<220>
<223> oligonucleotide primer

<400> 2
acttggcgtg tcctttgata gt 22

<210> 3
<211> 22
<212> DNA
<213> Artificial

<220>
<223> oligonucleotide primer

<400> 3
gagatccctc caaaatcaag tg 22

<210> 4
<211> 22
<212> DNA
<213> Artificial

<220>
<223> oligonucleotide primer

<400> 4
gtttttctag acggcaggtc ag 22

<210> 5
<211> 2091
<212> DNA
<213> Homo sapiens

<400> 5
gagcggagcc gcgggcggga gggcggacgg accgactgac ggtagggacg ggaggcgagc 60
aagatggcgc agacgcaggg cacccggagg aaagtctgtt actactacga cggggatgtt 120
ggaaattact attatggaca aggccaccca atgaagcctc accgaatccg catgactcat 180
aatttgctgc tcaactatgg tctctaccga aaaatggaaa tctatcgccc tcacaaagcc 240
aatgctgagg agatgaccaa gtaccacagc gatgactaca ttaaattctt gcgctccatc 300
cgtccagata acatgtcgga gtacagcaag cagatgcaga gattcaacgt tggtgaggac 360
tgtccagtat tcgatggcct gtttgagttc tgtcagttgt ctactggtgg ttctgtggca 420
agtgctgtga aacttaataa gcagcagacg gacatcgctg tgaattgggc tgggggcctg 480
caccatgcaa agaagtccga ggcatctggc ttctgttacg tcaatgatat cgtcttggcc 540
atcctggaac tgctaaagta tcaccagagg gtgctgtaca ttgacattga tattcaccat 600
ggtgacggcg tggaagaggc cttctacacc acggaccggg tcatgactgt gtcctttcat 660
aagtatggag agtacttccc aggaactggg gacctacggg atatcggggc tggcaaaggc 720
aagtattatg ctgttaacta cccgctccga gacgggattg atgacgagtc ctatgaggcc 780
attttcaagc cggtcatgtc caaagtaatg gagatgttcc agcctagtgc ggtggtctta 840
cagtgtggct cagactccct atctggggat cggttaggtt gcttcaatct aactatcaaa 900
ggacacgcca agtgtgtgga atttgtcaag agctttaacc tgcctatgct gatgctggga 960
ggcggtggtt acaccattcg taacgttgcc cggtgctgga catatgagac agctgtggcc 1020
ctggatacgg agatccctaa tgagcttcca tacaatgact actttgaata ctttggacca 1080
gatttcaagc tccacatcag tccttccaat atgactaacc agaacacgaa tgagtacctg 1140
gagaagatca aacagcgact gtttgagaac cttagaatgc tgccgcacgc acctggggtc 1200
caaatgcagg cgattcctga ggacgccatc cctgaggaga gtggcgatga ggacgaagac 1260
gaccctgaca agcgcatctc gatctgctcc tctgacaaac gaattgcctg tgaggaagag 1320
ttctccgatt ctgaagagga gggagagggg ggccgcaaga actcttccaa cttcaaaaaa 1380
gccaagagag tcaaaacaga ggatgaaaaa gagaaagacc cagaggagaa gaaagaagtc 1440
accgaagagg agaaaaccaa ggaggagaag ccagaagcca aaggggtcaa ggaggaggtc 1500
aagttggcct gaatggacct ctccagctct ggcttcctgc tgagtccctc acgtttcttc 1560
cccaacccct cagattttat attttctatt tctctgtgta tttatataaa aatttattaa 1620
atataaatat ccccagggac agaaaccaag gccccgagct cagggcagct gtgctgggtg 1680
agctcttcca ggagccacct tgccacccat tcttcccgtt cttaactttg aaccataaag 1740
ggtgccaggt ctgggtgaaa gggatacttt tatgcaacca taagacaaac tcctgaaatg 1800
ccaagtgcct gcttagtagc tttggaaagg tgcccttatt gaacattcta gaaggggtgg 1860
ctgggtcttc aaggatctcc tgtttttttc aggctcctaa agtaacatca gccattttta 1920
gattggttct gttttcgtac cttcccactg gcctcaagtg agccaagaaa cactgcctgc 1980

cctctgtctg tcttctccta attctgcagg tggaggttgc tagtctagtt tcctttttga 2040
gatactattt tcatttttgt gagcctcttt gtaataaaat ggtacatttc t 2091

<210> 6
<211> 482
<212> PRT
<213> Homo sapiens

<400> 6

Met Ala Gln Thr Gln Gly Thr Arg Arg Lys Val Cys Tyr Tyr Tyr Asp
1               5                   10                  15

Gly Asp Val Gly Asn Tyr Tyr Tyr Gly Gln Gly His Pro Met Lys Pro
            20                  25                  30

His Arg Ile Arg Met Thr His Asn Leu Leu Leu Asn Tyr Gly Leu Tyr
        35                  40                  45

Arg Lys Met Glu Ile Tyr Arg Pro His Lys Ala Asn Ala Glu Glu Met
    50                  55                  60

Thr Lys Tyr His Ser Asp Asp Tyr Ile Lys Phe Leu Arg Ser Ile Arg
65                  70                  75                  80

Pro Asp Asn Met Ser Glu Tyr Ser Lys Gln Met Gln Arg Phe Asn Val
                85                  90                  95

Gly Glu Asp Cys Pro Val Phe Asp Gly Leu Phe Glu Phe Cys Gln Leu
            100                 105                 110

Ser Thr Gly Gly Ser Val Ala Ser Ala Val Lys Leu Asn Lys Gln Gln
        115                 120                 125

Thr Asp Ile Ala Val Asn Trp Ala Gly Gly Leu His His Ala Lys Lys
    130                 135                 140

Ser Glu Ala Ser Gly Phe Cys Tyr Val Asn Asp Ile Val Leu Ala Ile
145                 150                 155                 160

Leu Glu Leu Leu Lys Tyr His Gln Arg Val Leu Tyr Ile Asp Ile Asp
                165                 170                 175

Ile His His Gly Asp Gly Val Glu Glu Ala Phe Tyr Thr Thr Asp Arg
            180                 185                 190

Val Met Thr Val Ser Phe His Lys Tyr Gly Glu Tyr Phe Pro Gly Thr
        195                 200                 205

Gly Asp Leu Arg Asp Ile Gly Ala Gly Lys Gly Lys Tyr Tyr Ala Val
    210                 215                 220

Asn Tyr Pro Leu Arg Asp Gly Ile Asp Asp Glu Ser Tyr Glu Ala Ile
225                 230                 235                 240

Phe Lys Pro Val Met Ser Lys Val Met Glu Met Phe Gln Pro Ser Ala
                245                 250                 255

Val Val Leu Gln Cys Gly Ser Asp Ser Leu Ser Gly Asp Arg Leu Gly
            260                 265                 270

Cys Phe Asn Leu Thr Ile Lys Gly His Ala Lys Cys Val Glu Phe Val
        275                 280                 285

Lys Ser Phe Asn Leu Pro Met Leu Met Leu Gly Gly Gly Gly Tyr Thr
    290                 295                 300

Ile Arg Asn Val Ala Arg Cys Trp Thr Tyr Glu Thr Ala Val Ala Leu
305                 310                 315                 320

Asp Thr Glu Ile Pro Asn Glu Leu Pro Tyr Asn Asp Tyr Phe Glu Tyr
                325                 330                 335

Phe Gly Pro Asp Phe Lys Leu His Ile Ser Pro Ser Asn Met Thr Asn
            340                 345                 350

Gln Asn Thr Asn Glu Tyr Leu Glu Lys Ile Lys Gln Arg Leu Phe Glu
        355                 360                 365

Asn Leu Arg Met Leu Pro His Ala Pro Gly Val Gln Met Gln Ala Ile
    370                 375                 380

Pro Glu Asp Ala Ile Pro Glu Glu Ser Gly Asp Glu Asp Glu Asp Asp
385                 390                 395                 400

Pro Asp Lys Arg Ile Ser Ile Cys Ser Ser Asp Lys Arg Ile Ala Cys
                405                 410                 415

Glu Glu Glu Phe Ser Asp Ser Glu Glu Glu Gly Glu Gly Gly Arg Lys
            420                 425                 430

Asn Ser Ser Asn Phe Lys Lys Ala Lys Arg Val Lys Thr Glu Asp Glu
        435                 440                 445

Lys Glu Lys Asp Pro Glu Glu Lys Lys Glu Val Thr Glu Glu Glu Lys
    450                 455                 460

Thr Lys Glu Glu Lys Pro Glu Ala Lys Gly Val Lys Glu Glu Val Lys
465                 470                 475                 480

Leu Ala

<210> 7
<211> 2016
<212> DNA
<213> Homo sapiens

<400> 7
gagcggagcc gcgggcggga gggcggacgg accgactgac ggtagggacg ggaggcgagc 60

aagatggcgc agacgcaggg cacccggagg aaagtctgtt actactacga cggggatgtt 120

ggaaattact attatggaca aggccaccca atgaagcctc accgaatccg catgactcat 180

aatttgctgc tcaactatgg tctctaccga aaaatggaaa tctatcgccc tcacaaagcc 240

aatgctgagg agatgaccaa gtaccacagc gatgactaca ttaaattctt gcgctccatc 300

cgtccagata acatgtcgga gtacagcaag cagatgcaga gatcaagtgc tgtgaaactt 360

aataagcagc agacggacat cgctgtgaat tgggctgggg gcctgcacca tgcaaagaag 420

tccgaggcat ctggcttctg ttacgtcaat gatatcgtct tggccatcct ggaactgcta 480

aagtatcacc agagggtgct gtacattgac attgatattc accatggtga cggcgtggaa 540

gaggccttct acaccacgga ccgggtcatg actgtgtcct ttcataagta tggagagtac 600

ttcccaggaa ctggggacct acgggatatc ggggctggca aaggcaagta ttatgctgtt 660

aactacccgc tccgagacgg gattgatgac gagtcctatg aggccatttt caagccggtc 720

atgtccaaag taatggagat gttccagcct agtgcggtgg tcttacagtg tggctcagac 780

tccctatctg gggatcggtt aggttgcttc aatctaacta tcaaaggaca cgccaagtgt 840

gtggaatttg tcaagagctt taacctgcct atgctgatgc tgggaggcgg tggttacacc 900

attcgtaacg ttgcccggtg ctggacatat gagacagctg tggccctgga tacggagatc 960

cctaatgagc ttccatacaa tgactacttt gaatactttg gaccagattt caagctccac 1020

atcagtcctt ccaatatgac taaccagaac acgaatgagt acctggagaa gatcaaacag 1080

cgactgtttg agaaccttag aatgctgccg cacgcacctg gggtccaaat gcaggcgatt 1140

cctgaggacg ccatccctga ggagagtggc gatgaggacg aagacgaccc tgacaagcgc 1200

atctcgatct gctcctctga caaacgaatt gcctgtgagg aagagttctc cgattctgaa 1260

gaggagggag aggggggccg caagaactct tccaacttca aaaaagccaa gagagtcaaa 1320

acagaggatg aaaaagagaa agacccagag gagaagaaag aagtcaccga agaggagaaa 1380

accaaggagg agaagccaga agccaaaggg gtcaaggagg aggtcaagtt ggcctgaatg 1440

gacctctcca gctctggctt cctgctgagt ccctcacgtt tcttccccaa cccctcagat 1500

tttatatttt ctatttctct gtgtatttat ataaaaattt attaaatata aatatcccca 1560

gggacagaaa ccaaggcccc gagctcaggg cagctgtgct gggtgagctc ttccaggagc 1620

caccttgcca cccattcttc ccgttcttaa ctttgaacca taaagggtgc caggtctggg 1680

tgaaagggat acttttatgc aaccataaga caaactcctg aaatgccaag tgcctgctta 1740

gtagctttgg aaaggtgccc ttattgaaca ttctagaagg ggtggctggg tcttcaagga 1800

tctcctgttt ttttcaggct cctaaagtaa catcagccat ttttagattg gttctgtttt 1860

cgtaccttcc cactggcctc aagtgagcca agaaacactg cctgccctct gtctgtcttc 1920
tcctaattct gcaggtggag gttgctagtc tagtttcctt tttgagatac tattttcatt 1980
tttgtgagcc tctttgtaat aaaatggtac atttct 2016

<210> 8
<211> 211
<212> PRT
<213> Homo sapiens

<400> 8

Met Ala Gln Thr Gln Gly Thr Arg Arg Lys Val Cys Tyr Tyr Tyr Asp
1               5                   10                  15

Gly Asp Val Gly Asn Tyr Tyr Tyr Gly Gln Gly His Pro Met Lys Pro
            20                  25                  30

His Arg Ile Arg Met Thr His Asn Leu Leu Leu Asn Tyr Gly Leu Tyr
        35                  40                  45

Arg Lys Met Glu Ile Tyr Arg Pro His Lys Ala Asn Ala Glu Glu Met
    50                  55                  60

Thr Lys Tyr His Ser Asp Asp Tyr Ile Lys Phe Leu Arg Ser Ile Arg
65                  70                  75                  80

Pro Asp Asn Met Ser Glu Tyr Ser Lys Gln Met Gln Arg Ser Ser Ala
                85                  90                  95

Val Lys Leu Asn Lys Gln Gln Thr Asp Ile Ala Val Asn Trp Ala Gly
            100                 105                 110

Gly Leu His His Ala Lys Lys Ser Glu Ala Ser Gly Phe Cys Tyr Val
        115                 120                 125

Asn Asp Ile Val Leu Ala Ile Leu Glu Leu Leu Lys Tyr His Gln Arg
    130                 135                 140

Val Leu Tyr Ile Asp Ile Asp Ile His His Gly Asp Gly Val Glu Glu
145                 150                 155                 160

Ala Phe Tyr Thr Thr Asp Arg Val Met Thr Val Ser Phe His Lys Tyr
                165                 170                 175

Gly Glu Tyr Phe Pro Gly Thr Gly Asp Leu Arg Asp Ile Gly Ala Gly
            180                 185                 190

Lys Gly Lys Tyr Tyr Ala Val Asn Tyr Pro Leu Arg Asp Gly Ile Asp
        195                 200                 205

Asp Glu Ser
    210

<210> 9
<211> 2124
<212> DNA
<213> Homo sapiens

<400> 9
   ctcccccctg ggtcggacgc tgagcggagc cgcgggcggg agggcggacg gaccgactga 60
   cggtagggac gggaggcgag caagatggcg cagacgcagg gcacccggag gaaagtctgt 120
   tactactacg acggggatgt tggaaattac tattatggac aaggccaccc aatgaagcct 180
   caccgaatcc gcatgactca taatttgctg ctcaactatg gtctctaccg aaaaatggaa 240
   atctatcgcc ctcacaaagc caatgctgag gagatgacca agtaccacag cgatgactac 300
   attaaattct tgcgctccat ccgtccagat aacatgtcgg agtacagcaa gcagatgcag 360
   agattcaacg ttggtgagga ctgtccagta ttcgatggcc tgtttgagtt ctgtcagttg 420
   tctactggtg gttctgtggc aagtgctgtg aaacttaata agcagcagac ggacatcgct 480
   gtgaattggg ctgggggcct gcaccatgca aagaagtccg aggcatctgg cttctgttac 540
   gtcaatgata tcgtcttggc catcctggaa ctgctaaagt atcaccagag ggtgctgtac 600
   attgacattg atattcacca tggtgacggc gtggaagagg ccttctacac cacggaccgg 660
   gtcatgactg tgtcctttca taagtatgga gagtacttcc caggaactgg ggacctacgg 720
   gatatcgggg ctggcaaagg caagtattat gctgttaact acccgctccg agacgggatt 780
   gatgacgagt cctatgaggc cattttcaag ccggtcatgt ccaaagtaat ggagatgttc 840
   cagcctagtg cggtggtctt acagtgtggc tcagactccc tatctgggga tcggttaggt 900
   tgcttcaatc taactatcaa aggacacgcc aagtgtgtgg aatttgtcaa gagctttaac 960
   ctgcctatgc tgatgctggg aggcggtggt tacaccattc gtaacgttgc ccggtgctgg 1020
   acatatgaga cagctgtggc cctggatacg gagatcccta atgagcttcc atacaatgac 1080
   tactttgaat actttggacc agatttcaag ctccacatca gtccttccaa tatgactaac 1140
   cagaacacga atgagtacct ggagaagatc aaacagcgac tgtttgagaa ccttagaatg 1200
   ctgccgcacg cacctggggt ccaaatgcag gcgattcctg aggacgccat ccctgaggag 1260
   agtggcgatg aggacgaaga cgaccctgac aagcgcatct cgatctgctc ctctgacaaa 1320
   cgaattgcct gtgaggaaga gttctccgat tctgaagagg agggagaggg gggccgcaag 1380
   aactcttcca acttcaaaaa agccaagaga gtcaaaacag aggatgaaaa agagaaagac 1440
   ccagaggaga agaaagaagt caccgaagag gagaaaacca aggaggagaa gccagaagcc 1500
   aaaggggtca aggaggaggt caagttggcc tgaatggacc tctccagctc tggcttcctg 1560
   ctgagtccct cacgtttctt ccccaacccc tcagatttta tattttctat ttctctgtgt 1620
   atttatataa aaatttatta aatataaata tccccaggga cagaaaccaa ggccccgagc 1680
   tcagggcagc tgtgctgggt gagctcttcc aggagccacc ttgccaccca ttcttcccgt 1740
   tcttaacttt gaaccataaa gggtgccagg tctgggtgaa agggatactt ttatgcaacc 1800
   ataagacaaa ctcctgaaat gccaagtgcc tgcttagtag ctttggaaag gtgcccttat 1860
   tgaacattct agaaggggtg gctgggtctt caaggatctc ctgttttttt caggctccta 1920
   aagtaacatc agccattttt agattggttc tgttttcgta ccttcccact ggcctcaagt 1980
   gagccaagaa acactgcctg ccctctgtct gtcttctcct aattctgcag gtggaggttg 2040
   ctagtctagt ttcctttttg agatactatt ttcatttttg tgagcctctt tgtaataaaa 2100
   tggtacattt ctatatcctc ctga 2124

<210> 10
<211> 482
<212> PRT
<213> Homo sapiens

<400> 10

Met Ala Gln Thr Gln Gly Thr Arg Arg Lys Val Cys Tyr Tyr Tyr Asp
1               5                   10                  15

Gly Asp Val Gly Asn Tyr Tyr Tyr Gly Gln Gly His Pro Met Lys Pro
            20                  25                  30

His Arg Ile Arg Met Thr His Asn Leu Leu Leu Asn Tyr Gly Leu Tyr
        35                  40                  45

Arg Lys Met Glu Ile Tyr Arg Pro His Lys Ala Asn Ala Glu Glu Met
    50                  55                  60

Thr Lys Tyr His Ser Asp Asp Tyr Ile Lys Phe Leu Arg Ser Ile Arg
65                  70                  75                  80

Pro Asp Asn Met Ser Glu Tyr Ser Lys Gln Met Gln Arg Phe Asn Val
                85                  90                  95

Gly Glu Asp Cys Pro Val Phe Asp Gly Leu Phe Glu Phe Cys Gln Leu
            100                 105                 110

Ser Thr Gly Gly Ser Val Ala Ser Ala Val Lys Leu Asn Lys Gln Gln
        115                 120                 125

Thr Asp Ile Ala Val Asn Trp Ala Gly Gly Leu His His Ala Lys Lys
    130                 135                 140

Ser Glu Ala Ser Gly Phe Cys Tyr Val Asn Asp Ile Val Leu Ala Ile
145                 150                 155                 160

Leu Glu Leu Leu Lys Tyr His Gln Arg Val Leu Tyr Ile Asp Ile Asp
                165                 170                 175

Ile His His Gly Asp Gly Val Glu Glu Ala Phe Tyr Thr Thr Asp Arg
            180                 185                 190

Val Met Thr Val Ser Phe His Lys Tyr Gly Glu Tyr Phe Pro Gly Thr
        195                 200                 205

Gly Asp Leu Arg Asp Ile Gly Ala Gly Lys Gly Lys Tyr Tyr Ala Val
    210                 215                 220

Asn Tyr Pro Leu Arg Asp Gly Ile Asp Asp Glu Ser Tyr Glu Ala Ile
225                 230                 235                 240

Phe Lys Pro Val Met Ser Lys Val Met Glu Met Phe Gln Pro Ser Ala
                245                 250                 255

Val Val Leu Gln Cys Gly Ser Asp Ser Leu Ser Gly Asp Arg Leu Gly
            260                 265                 270

Cys Phe Asn Leu Thr Ile Lys Gly His Ala Lys Cys Val Glu Phe Val
        275                 280                 285

Lys Ser Phe Asn Leu Pro Met Leu Met Leu Gly Gly Gly Gly Tyr Thr
    290                 295                 300

Ile Arg Asn Val Ala Arg Cys Trp Thr Tyr Glu Thr Ala Val Ala Leu
305                 310                 315                 320

Asp Thr Glu Ile Pro Asn Glu Leu Pro Tyr Asn Asp Tyr Phe Glu Tyr
                325                 330                 335

Phe Gly Pro Asp Phe Lys Leu His Ile Ser Pro Ser Asn Met Thr Asn
            340                 345                 350

Gln Asn Thr Asn Glu Tyr Leu Glu Lys Ile Lys Gln Arg Leu Phe Glu
        355                 360                 365

Asn Leu Arg Met Leu Pro His Ala Pro Gly Val Gln Met Gln Ala Ile
    370                 375                 380

Pro Glu Asp Ala Ile Pro Glu Glu Ser Gly Asp Glu Asp Glu Asp Asp
385                 390                 395                 400

Pro Asp Lys Arg Ile Ser Ile Cys Ser Ser Asp Lys Arg Ile Ala Cys
                405                 410                 415

Glu Glu Glu Phe Ser Asp Ser Glu Glu Glu Gly Glu Gly Gly Arg Lys
            420                 425                 430

Asn Ser Ser Asn Phe Lys Lys Ala Lys Arg Val Lys Thr Glu Asp Glu
        435                 440                 445

Lys Glu Lys Asp Pro Glu Glu Lys Lys Glu Val Thr Glu Glu Glu Lys
    450                 455                 460

Thr Lys Glu Glu Lys Pro Glu Ala Lys Gly Val Lys Glu Glu Val Lys
465                 470                 475                 480

Leu Ala

<210>  11
<211>  29
<212>  DNA
<213>  Artificial

<220>
<223>  oligonucleotide primer

<400>  11
cagagctgct aacaggaggc ggaggcgga                                        29

<210>  12
<211>  21
<212>  DNA
<213>  Artificial

<220>
<223>  oligonucleotide primer

<400>  12
catagtagta acggagggcg c                                                21

<210>  13
<211>  22
<212>  DNA
<213>  Artificial

<220>
<223>  oligonucleotide primer

<400>  13
ttgtggtggg aaggggatgt tt                                               22

<210>  14
<211>  20
<212>  DNA
<213>  Artificial

<220>
<223>  oligonucleotide primer

<400>  14
cgaagtccgt ctgttcctgt                                                  20

## Description

### FIELD OF THE INVENTION

This invention relates to a method for treating ERG-positive prostate cancer patients with an agent affecting one or more ERG-associated genes and/or manipulating of one or more ERG-related pathways, optionally in combination with an androgen deprivation therapy. Furthermore, the invention concerns a methods for screening prostate cancer patients which may benefit from said treatment, assessing the efficacy of a therapy for treating prostate cancer in a patient, assessing progression of prostate cancer in a patient, selecting an agent to be tested for usefulness in the treatment of prostate cancer, and for assessing prostate carcinogenic potential of an agent.

### BACKGROUND OF THE INVENTION

The publications and other materials used herein are intended to illuminate the background of the invention, and in particular, cases to provide additional details respecting the practice, and are incorporated by reference.

Fusion transcripts arising from translocations of a prostate-specific *TMPRSS2* gene with oncogenic ETS factors *ERG, ETV1* or *ETV4* were recently identified in up to half of all human prostate cancers (1, 2). Although both translocation partners have been previously studied in prostate cancers (3, 4), the identification of a translocation between the two genes provides new insights into the mechanisms of androgen-dependent prostate tumorigenesis. *TMPRSS2* encodes for a transmembrane serine protease which is strongly expressed in both normal and prostate cancer tissues, and regulated by androgens. ERG, ETV1 and ETV4 belong to the ETS transcription factor family characterized by a conserved DNA binding domain. ETS proteins have been previously implicated in oncogenic translocations in Ewing's sarcomas, leukemias, lymphomas, fibrosarcomas and secretory breast carcinomas (5, 6, 7). ETS factors regulate expression of genes with important cancer-relevant biological processes, such as cell growth, differentiation and transformation (5). However, the specific roles of particular ETS factors in prostate cancer development and progression have remained unclear, and in particular the molecular consequences of the *TMPRSS2: ERG* fusion gene formation remain unknown. Here, we have explored the role of ETS factor alterations in prostate cancers, and investigated the genetic mechanisms causing oncogenic ETS gene fusions. Furthermore, we have identified potential downstream pathways and gene signatures (= genes that are co-expressed with oncogenic ERG) that are associated with the ETS activation.

### OBJECTS AND SUMMARY OF THE INVENTION

ERG-translocation with the TMPRSS2 androgen-responsive promoter elements is
a pathogenetically important, recently described genetic alteration in prostate cancer, which may be causally contributing to prostate cancer. In physiological situations, androgen receptor (AR) binds to androgen responsive elements in the DNA, activating or silencing
androgen-dependent genes. Following ERG translocation, oncogenic effects arise so that androgen and AR effects are mediated through ERG downstream target genes. While the general concept has been previously presented, the actual mechanisms of ERG-associated pathogenesis in the prostate are still unknown, and the target genes and pathways affected by ERG action in human prostate cancer cells remain unclear.

In the study shown below, we define a set of 55 ERG associated genes and a number of pathways that may mediate or that are associated with the ERG-activated prostate cancers. This set of genes and the biological processes and pathways where they are active may be useful for prostate cancer diagnosis or therapy. This kind of gene sets are often called a signature, fingerprint, profile, or a marker panel and they may be useful individually or in any combinations of the genes. Each of these ERG associated genes defines a diagnostic opportunity or can guide the selection of therapy or serve as a biomarker for the efficacy of ERG-therapy or ERG-associated therapy.

Recent discovery by US. investigators of ERG-gene translocations in prostate cancer has shed new light onto the pathogenesis of the disease. This may be one of the most important cancer genetics discoveries in the past 30 years, as it may be involved in up close to 50% of all prostate cancers. We have now taken the first steps to shed light into the mechanism of disease in ERG-positive prostate cancers by discovering that, for example, HDAC1 gene and epigenetic gene regulation may be involved. This points to targeted therapeutic possibilities.

Thus, according to one aspect, this invention concerns a method for screening of prostate cancer patients with ERG-activation or ERG-translocation in order to evaluate said patients' response to an anti-ERG therapy, optionally in combination with an androgen deprivation therapy, said method being based on use one or more ERG-associated genes and/or one or more ERG-related pathways as a biomarker. An altered level of expression of an ERG-associated gene in a patient sample, compared to a control sample taken from the same patient or from a control subject, indicates that the prostate cancer has arisen due to ERG activation and that the patient is likely to benefit from an anti-ERG therapy.

According to another aspect, the invention concerns the use of an agent i) inactivating, stimulating or altering the expression of an ERG-associated gene or protein in a prostate cancer patient, and/or ii) manipulating an ERG-related pathway in said patient, optionally in combination with an agent reducing the androgen level in said patient, for the manufacture of a pharmaceutical composition useful for treatment of prostate cancer in a patient with confirmed ERG-activation or ERG-translocation.

According to a third aspect, the invention concerns a method for treatment of prostate cancer in a patient with confirmed ERG-activation or ERG-translocation, said method comprising administering of an effective amount of an agent
i) inactivating, stimulating or altering the expression of an ERG-associated gene or protein in said patient, and/or
ii) manipulating an ERG-related pathway in said patient,
   and optionally administering an effective amount of an agent reducing the androgen level in said patient.

According to a fourth aspect, the invention concerns an agent i) inactivating, stimulating or altering the expression of an ERG-associated gene or protein in a prostate cancer patient, and/or ii) manipulating an ERG-related pathway in said patient, optionally in combination with an agent reducing the androgen level in said patient, for treatment of prostate cancer in a patient with confirmed ERG-activation or ERG-translocation.

According to a fifth aspect, the invention concerns a method for assessing the efficacy of a therapy for treating prostate cancer in a patient, said method comprising comparing expression of at least one biomarker, which is an ERG-associated gene and/or an ERG-related pathway, in a first sample obtained from the patient prior to providing at least a portion of said therapy to the patient, and the expression of said biomarker or biomarkers in a second sample obtained from the patient at a later stage of said therapy. A reversed level of expression of the marker or markers in the second sample relative to that in the first sample is an indication that the therapy is efficacious for inhibiting prostate cancer in patients.

According to a sixth aspect, the invention concerns a method for assessing progression of prostate cancer in a patient, comprising the steps of: a) detecting in a sample from the patient at a first time point, the expression of a biomarker, which is an ERG-associated gene and/or an ERG-related pathway,
b) repeating the detection of expression of said biomarker at a subsequent time point in time, and
c) comparing the level of expression detected in the first and second detection steps, thereby monitoring the progression of prostate cancer in the patient. If the biomarker monitored is an upregulated ERG-associated gene, an increased level of expression of the marker in the sample at the subsequent time point from that of the sample at the first time point is an indication that the prostate cancer has progressed in the patient, whereas a decreased level of expression is an indication that the prostate cancer has regressed.

According to a seventh aspect, the invention concerns a method for selecting an agent to be tested for usefulness in the treatment of prostate cancer, said method comprising the steps of
a) dividing a sample, drawn from the patient and/or comprising prostate cancer cells, in aliquots,
b) separately maintaining all sample aliquots in the presence of different test agents,
c) comparing the expression of at least one biomarker, which is an ERG-associated gene and/or an ERG-related pathway, in each of the aliquots,
   and
d) selecting as agent one that reverses the expression of said biomarker.

According to an eight aspect, the invention concerns a method for assessing the prostate carcinogenic potential of an agent, said method comprising the steps of
a) maintaining separate aliquots of prostate cells in the presence or absence of an agent, the carcinogenic potential of which is to be tested, and
b) comparing expression of a biomarker, which is an ERG-associated gene and/or an ERG-related pathway, in each of the aliquots, and
c) using an altered level of expression of said biomarker maintained in the presence of said agent, relative to that of the aliquot maintained in the absence of said agent, is an indication that the agent possesses prostate carcinogenic potential.

### BRIEF DESCRIPTION OF THE DRAWINGS

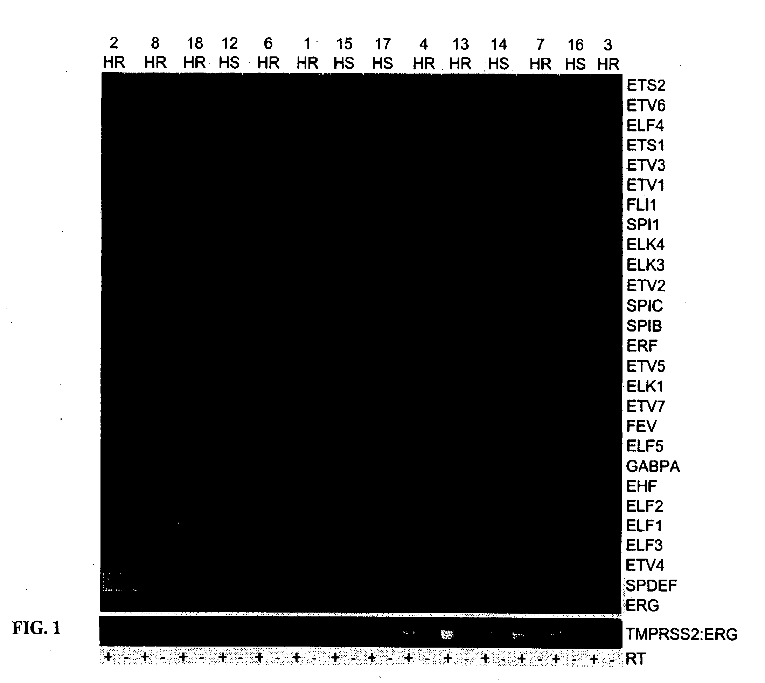

**Figure 1****.** Expression of 27 ETS family transcription factors in prostate tumors and metastases. Sample numbers together with either hormone sensitive (HS) or hormone refractory (HR) status of the specimens are indicated at the top of the heat-map. Each cell in the image shows the log2 expression ratio for the particular gene divided by the median expression of that gene in all samples. The ETS factors ERG, ETV4 and ETV1, known to be fused with TMPRSS2 in a subset of prostate cancers are highlighted in bold. Red indicates expression above the median. RT-PCR results to detect *TMPRSS2:ERG* fusions in the corresponding samples (+) together with the reverse transcriptase-negative controls (-) are shown at the bottom of the figure.

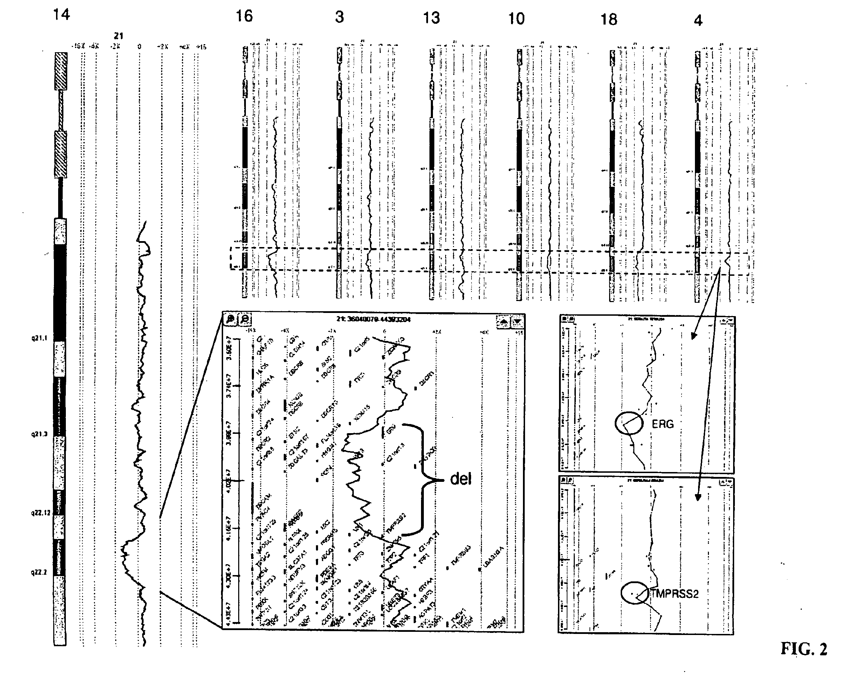

**Figure 2****.** Array-based CGH data showing deletions between *ERG* and *TMPRSS2* in advanced prostate cancers and metastases. Chromosome 21 copy number profiles at *ERG*/*TMPRSS2* region at q22.2-q22.3 are shown from specimens with reduction of copy number. In sample no 10, only a modest indication of copy number reduction was detected, whereas in sample18, the actual breakpoint occurred proximally from ERG. Examples from cases with interstitial deletion (sample no 14) and smaller microdeletions affecting ERG and TMPRSS2 loci (sample no 4) are also shown.

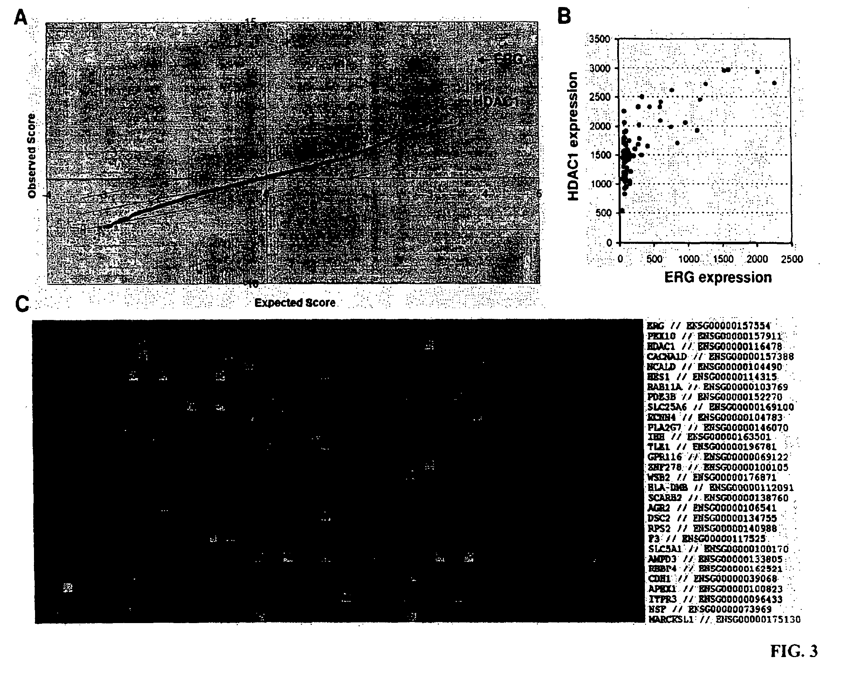

**Figure 3****.** Identification of ERG associated genes in prostate cancer. (A) Scatter plot of the observed relative difference score (x-axis) versus the expected relative difference score (y-axis) from the comparison of ERG positive prostate cancers with ERG negative prostate cancers by SAM. ERG and HDAC1 are indicated in the figure. The gene with the second highest observed d-score is PEX10, which was not found among the top correlating genes with ERG in the other data sets analyzed and is therefore not further discussed. (B) Co-expression plot of ERG and HDAC1 in prostate cancer samples used in SAM. Red indicates samples with high ERG expression, black samples with low ERG expression. (C) Heat-map of the SAM positive hits. The most highly correlated genes with ERG are indicated.

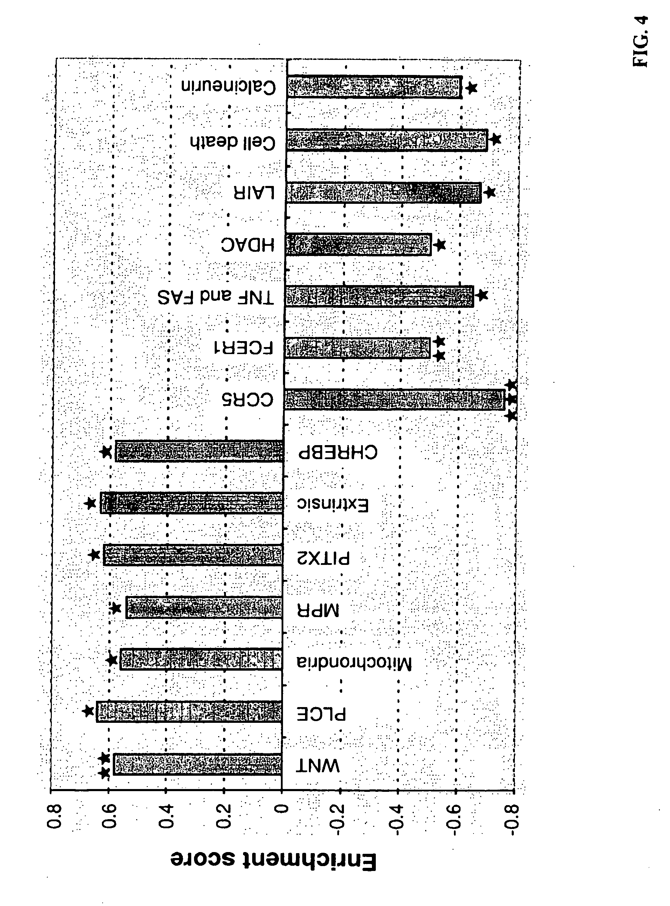

Figure 4. Gene sets showing significant enrichment among ERG positive prostate cancers. Both the upregulated (nominal p-value <0.03, enrichment score >0.5) and downregulated (nominal p-value <0.03, enrichment score <-0.5) gene sets in ERG positive prostate cancers identified by GSEA analysis are shown in the figure. Enrichment score is plotted in the y-axis. Stars indicate the significance of the enrichment score (* p<0.05, ** p<0.01, *** p<0.001).

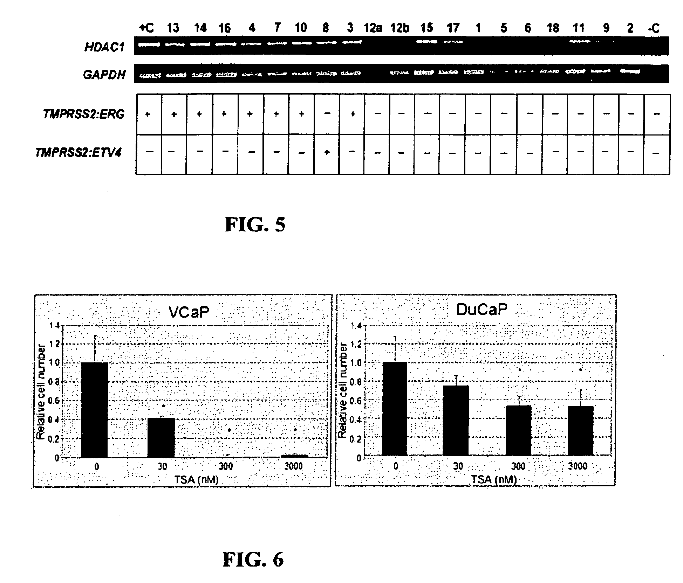

Figure 5. Semi-quantitative RT-PCR analysis of advanced prostate cancer samples for the *HDAC1* and *GAPDH* expression levels. Briefly, the 19 advanced prostate cancer cDNAs prepared for the *TMPRSS2: ETS* fusion identification were used here to analyze *HDAC1* cDNA (primers: 5'-AAGTATCACCAGAGGGTGCTG'T-3' (SEQ ID NO. I) and 5'-ACTTGGCGTGTCCTTTGATAGT-3') (SEQ ID NO. 2) and *GAPDH* cDNA (primers: 5'-GAGATCCCTCCAAAATCAAGTG-3' (SEQ ID NO. 3) and 5'-GTTTTTCTAGACGGCAGGTCAG-3') (SEQ ID NO. 4) levels by PCR amplification. The presence of *TMPRSS2-ERG* and *TMPRSS2-ETV4* fusion transcripts within samples are presented in the table. The DuCaP prostate cancer cell cDNA was used as a positive control (+C) and a blank with no template as a negative control (-C).
Figure 6. Analysis of the effect of Trichostatin A (TSA) treatment on VCaP and DuCaP prostate cancer cell number by using cell titer blue assay. Briefly, cells were plated in low concentration on 384-well plates (2000 cells/well) and let to attach overnight. Drug containing medium was added and CTB assay measurement with Envision Plate reader were done after 48 h incubation. Both cell lines show clear dose dependent growth inhibition in response to TSA. For drawing the figure, cell culture medium only containing well intensities were extracted from actual measurements. Each data point is represented by the mean value of three independent measurements (mean + S.D.).
Drug concentrations are presented on x-axis and relative cell number (control level set to 1) on y-axis. Statistical significance (p<0.5) of the differences between cell amounts in treated wells in comparison to untreated control wells is indicated with star using Student's t-test assuming that the data is unpaired and variance is unequal.

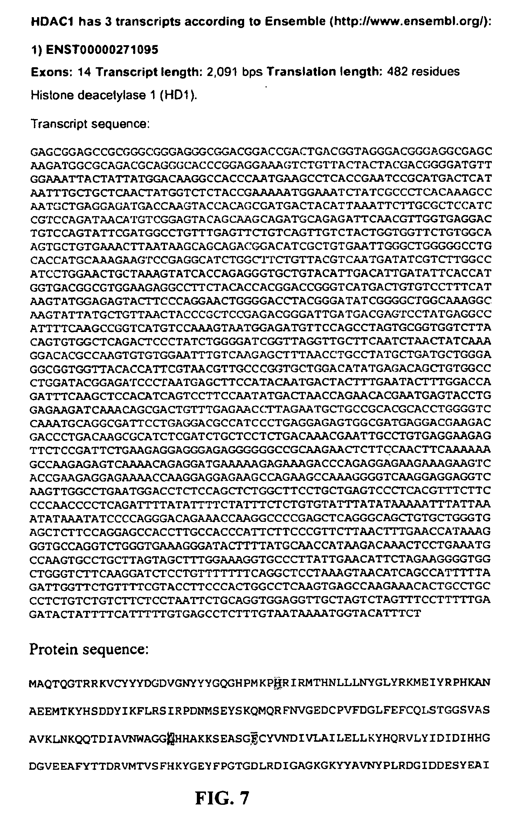

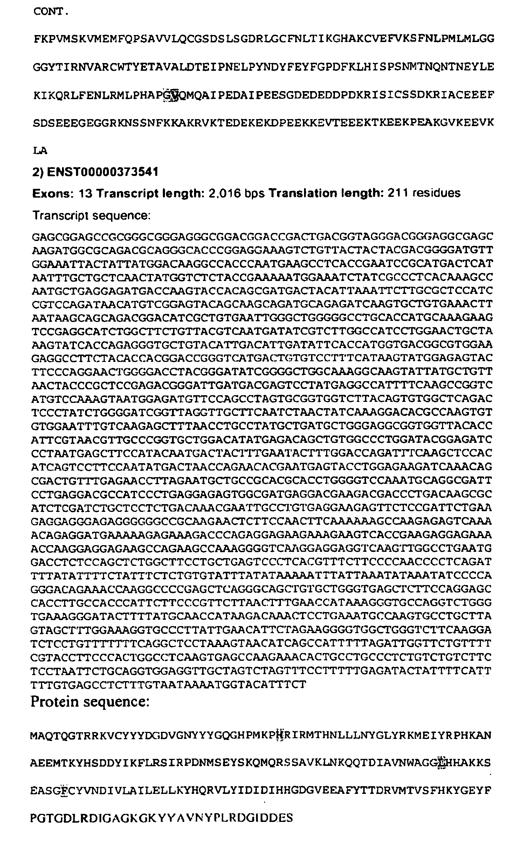

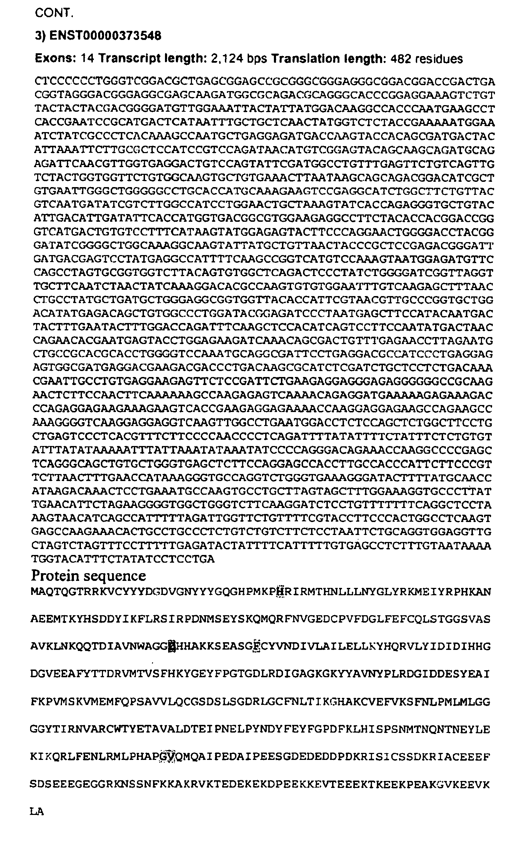

**Figure 7** shows 3 transcripts (cDNA and the corresponding amino acid sequence) of HDAC1 according to Ensemble (http://www.ensembl.org/). The TMPRSS2 gene can be fused to the ERG gene in many different ways. Tomlins et al., 2005 (2) described the fusion for the first time:
   complete exon 1 of TMPRSS2 was fused with the beginning of exon 4 of ERG, or complete exon 1 of TMPRSS2 was fused with the beginning of exon 2 of ERG. Soller et al., 2006 (10) described new fusion transcripts where the exons 4 or 5 of the TMPRSS2 gene were fused to the exon 4 or 5 of ERG. The inventor of the present invention have additionally found two new fusions: complete exon 3 of TMPRSS2 fused with the beginning of exon 4 of ERG and a fusion where the complete exon 2 of TMPRSS2 was fused to 95 nucleotides that originate from the preceding intron 3 (genomic coordinates NC_000021: 38767914-38767820), followed by the beginning of exon 4 of ERG. Most likely, this sequence element represents a cryptic exon that is not or only rarely included in ERG transcripts. The sequences are listed in SEQ ID NO:s 5-10.
**Figure 8** shows the effect of the HDAC inhibitor TSA, 10 µM flutamide and their combination on VCaP prostate cancer cell proliferation (*P>0.01).
**Figure 9** shows the effect of the HDAC inhibitor MS-275, 10 µM flutamide and their combination on VCaP prostate cancer cell proliferation (*P>0.01).
**Figure 10** shows the effect of the HDAC inhibitor TSA, 10 µM flutamide and their combination on VCaP prostate cancer cell apoptosis measured by caspase-3 and -7 activity, compared to untreated control cells (*P>0.01).
**Figure 11** shows the effect of the HDAC inhibitor MS-275, 10 µM flutamide and their combination on VCaP prostate cancer cell apoptosis measured by caspase-3 and -7 activity, compared to untreated control cells (*P>0.01).
**Figure 12** shows that treatment with the HDAC inhibitor TSA reverses the pathways associated to ERG-positive prostate tumors in ERG-positive prostate cancer cell line VCaP (nominal p-value <0.001).
**Figure 13** shows that treatment with the HDAC inhibitor MS-275 reverses the pathways associated to ERG-positive prostate tumors in ERG-positive prostate cancer cell line VCaP (nominal p-value <0.001).

### DETAILED DESCRIPTION OF THE INVENTION

### Definitions:

The term "ERG-positive" refers especially to prostate cancer patients which are carriers of the TMPRSS2-ERG fusion gene. Additionally, cases with fusion events to other ETS factors such as ETV1 and ETV4 may be considered as similar, based on the related function of those ETS factors.

The term "ERG-associated gene" refers particularly to those mentioned in Table 3, but we stress that the term is not restricted to these examples.

The term "ERG-related pathways" refers particularly to those shown in Figure 4. However, the term is not restricted to those shown in Figure 4.

A brief explanation of some of the abbreviated terms appearing in this text is found in Table 7 at the end of the description. Other abbreviations are found in the HUGO database.

The term "anti-ERG therapy" shall be understood to cover counteracting the influence of one or more ERG-associated genes and/or manipulating one or more ERG-related pathways.

The term "androgen deprivation therapy" shall be understood to cover any therapy aimed to reduce the androgen level in the patient.

The tem "treatment" or "treating" shall be understood to include complete curing of the disease as well as amelioration or alleviation of the disease. The term shall also be understood to cover "prevention" including complete prevention, prophylaxis, as well as lowering the individual's risk of falling ill with the disease.

### Methods of counteracting the influence of an ERG-associated gene:

According to one embodiment, the influence of an ERG-associated gene can be counteracted by administering of an agent inactivating or stimulating the protein expressed by said ERG-associated gene by an inhibitor or activator, which is a small molecule or peptide. As examples of other agents inactivating the ERG-associated gene-expressed protein can be mentioned an antibody raised against said protein, or an aptamer (an oligonucleotide) affecting the protein conformation of said protein resulting in the inactivation of the same.

According to another preferable embodiment, the agent is an agent altering the expression of the ERG-associated gene. Such an agent can, for example, be a down regulating agent such as an antisense oligonucleotide, modified nucleotide, sequence of combination of different kinds of nucleotides to prevent or modify the protein synthesis. The antisense oligonucleotide can be a DNA molecule or an RNA molecule. The agent down regulating the expression of the ERG-associated gene can also be a small interfering RNA (siRNA), or a ribozyme complementary to a target region of the mRNA of the protein.

The term "complementary" means that a nucleotide sequence forms hydrogen bonds with the target RNA sequence by Watson-Crick or other base-pair interactions. The terms shall be understood to cover also sequences which are not 100 % complementary. It is believed that lower complementarity, even as low as 70 % or more, may work. However, 100 % complementarity is preferred.

The ribozyme technology is extensively described in the art. The following publications can be mentioned as examples: Ribozyme protocols: Turner, Philip C (editor) Humana Press, ISBN 0-89603-389-9, 512 pp. 1997.; Rossi JJ. Ribozymes, genomics and therapeutics. Chem Biol 6, R33-7, 1999.; and Ellington AD, Robertson MP, Bull J. Ribozymes in wonderland. Science 276, 546-7, 1997.

Also small interfering RNA molecules (siRNAs) would be useful. The application of siRNA:s has become important in the development of new therapies in the last years. O Heidenreich presents an overview of pharmaceutical applications in the article "Forging therapeutics from small interfering RNAs in European Pharmaceutical Review Issue 1, 2005. The principle has particularly been suggested for the treatment of tumors and carcinomas, sarcomas, hypercholesterolemia, neuroblastoma and herpetic stromal keratitis.

The principle of siRNA is extensively presented in literature. As examples can be mentioned the US patent publications 2003/0143732, 2003/0148507, 2003/0175950, 2003/0190635, 2004/0019001, 2005/0008617 and 2005/0043266. An siRNA duplex molecule comprises an antisense region and a sense strand wherein said antisense strand comprises sequence complementary to a target region in an mRNA sequence encoding a certain protein, and the sense strand comprises sequence complementary to the said antisense strand. Thus, the siRNA duplex molecule is assembled from two nucleic acid fragments wherein one fragment comprises the antisense strand and the second fragment comprises the sense strand of said siRNA molecule. The sense strand and antisense strand can be covalently connected via a linker molecule, which can be a polynucleotide linker or a non-nucleotide linker. The length of the antisense and sense strands are typically about 19 to 21 nucleotides each. Typically, the antisense strand and the sense strand both comprise a 3'-terminal overhang of a few, typically 2 nucleotides. The 5'-terminal of the antisense is typically a phosphate group (P). The siRNA duplexes having terminal phosphate groups (P) are easier to administrate into the cell than a single stranded antisense. In the cell, an active siRNA antisense strand is formed and it recognizes a target region of the target mRNA. This in turn leads to cleaving of the target RNA by the RISC endonuclease complex (RISC = RNA-induced silencing complex) and also in the synthesis of additional RNA by RNA dependent RNA polymerase (RdRP), which can activate DICER and result in additional siRNA duplex molecules, thereby amplifying the response.

The oligonucleotide (such as antisense, siRNA or ribozyme molecule) shall, when used as a pharmaceutical, be introduced in a target cell. The delivery can be accomplished in two principally different ways: 1) exogenous delivery of the oligonucleotide or 2) endogenous transcription of a DNA sequence encoding the oligonucleotide, where the DNA sequence is located in a vector.

Normal, unmodified RNA has low stability under physiological conditions because of its degradation by ribonuclease enzymes present in the living cell. If the oligonucleotide shall be administered exogenously, it is highly desirable to modify the molecule according to known methods so as to enhance its stability against chemical and enzymatic degradation.

Modifications of nucleotides to be administered exogenously in vivo are extensively described in the art. Principally, any part of the nucleotide, i.e the ribose sugar, the base and/or internucleotidic phosphodiester strands can be modified. For example, removal of the 2'-OH group from the ribose unit to give 2'-deoxyribosenucleotides results in improved stability. Prior discloses also other modifications at this group: the replacement of the ribose 2'-OH group with alkyl, alkenyl, allyl, alkoxyalkyl, halo, amino, azido or sulfhydryl groups. Also other modifications at the ribose unit can be performed: locked nucleid acids (LNA) containing methylene linkages between the 2'- and 4'-positions of the ribose can be employed to create higher intrinsic stability. Furthermore, the internucleotidic phosphodiester linkage can, for example, be modified so that one ore more oxygen is replaced by sulfur, amino, alkyl or alkoxy groups. Also the base in the nucleotides can be modified. Preferably, the oligonucleotide comprises modifications of one or more 2'-hydroxyl groups at ribose sugars, and/or modifications in one or more internucleotidic phosphodiester linkages, and/or one or more locked nucleic acid (LNA) modification between the 2'- and 4'-position of the ribose sugars. Particularly preferable modifications are, for example, replacement of one or more of the 2'-OH groups by 2'-deoxy, 2'-O-methyl, 2'-halo, eg. fluoro or 2'-methoxyethyl. Especially preferred are oligonucleotides where some of the internucleotide phoshodiester linkages also are modified, e.g. replaced by phosphorothioate linkages.

It should be stressed that the modifications mentioned above are only non-limiting examples.

### General examples of androgen deprivation therapies:

Androgen deprivation represents a treatment designed to suppress or block the production or subsequent downstream action of male sex hormones. Androgen deprivation (also called androgen ablation therapy or androgen suppression) can in principle be achieved by surgical removal of the testicles, or (in clinical practice) by taking drugs that act as antiandrogens. Antiandrogens are any drugs or compounds that block the production and metabolism of androgens, interfere with binding of androgens (testosterone or dihydro-testosterone, DHT) to the nuclear androgen receptor (AR), and thus inhibit the recognition of target DNA sequences by functional AR. As a result, proliferation, maintenance and tissue homeostasis of androgen-responsive tissues such as the prostate (incl. prostate cancer) is disturbed. Antiandrogens are routinely used in the treatment of prostate cancer. The antiandrogens most frequently used in clinical practice include flutamide (Eulexin), bicalutamide (Casodex), and nilutamide (Nilandron). However, upon anti-androgen treatment, patients frequently develop hormone-refractory progressed cancers that are considered as incurable. Hormone-refractory tumors may arise through a number of different genetic mechanisms, including the amplification of the AR gene on the X chromosome, or activating mutations that increase the spectrum and efficiency of activating ligands (such as other steroid hormones).

### General examples of methods for manipulating ERG-related pathways:

Pathways are biochemical reactions and interaction partners (sets of genes, proteins or enzymes) that constitute a coherent functional network in living cells. Metabolic pathways represent a sequence of enzymatic or other reactions by which one biological material is converted to another. **Signal transduction pathways** represent a sequence of enzymatic and other interactive events that convey a signal (e.g. a growth stimulus) from cell to cell, or from the outside to the inside of a cell. ERG and other ETS factors are involved in a number of overlapping signal transduction pathways that in general have the potential to stimulate cell proliferation, cell cycle progression, epigenetic silencing mechanisms, or to counteract terminal cell differentiation. Ectopic overexpression of ERG (and other ETS factors) in cancer cells is therefore expected to disturb or bypass a number of (overlapping) growth-regulatory mechanisms. Since pathways represent the true functional units of cellular systems, drugs and other compounds that interfere with different components within the same pathway may have similar or identical results, or the effects of multiple hits within one pathway or overlapping pathways may result in potentiated (synergistic) responses.

### Preferred biomarkers

Preferably, the ERG-associated gene is any of the genes listed in Table 3, or any combination of said genes. Even more preferably, the ERG-associated gene is any of the genes listed in Table 5, or any combination of said genes. From the experiments disclosed below shown in Table 5 it can be seen that certain ERG-associated genes that are upregulated and can be downregulated by treatment, while other ERG-associated genes that are down regulated and can be upregulated by treatment.

Preferable pathways disclosed in Table 4 and Figure 4. Particularly useful pathways may be WNT, TNF/FAS, apoptosis or HDAC pathway, or a combination thereof.

According to a particularly preferred embodiment, the ERG-associated gene is HDAC1 (Histone Deacetylase 1). Histone deacetylases (HDACs) are enzymes that affect gene transcription by selectively deacetylating ε-amino groups of several lysine residues on the core histone (and other) proteins. The organization and packaging of eukaryotic DNA are achieved through the addition of proteins, including the core histones H2A, H2B, H3 and H4, which together form the chromatin. The enzymatic modification of the core histones is of fundamental importance to conformational changes of the chromatin. The level of histone acetylation (as controlled by HDACs, and counteracting acetyl-transferases) strongly affects the transcriptional activity by inducing an open chromatin confirmation that allows the transcription machinery to more easily access promoters and enhancers. Chromatin acetylation at promoters correlates with transcriptional activity (euchromatin), whereas increased histone deacetylation correlates with gene silencing. High activity of HDACs has been closely associated with cell proliferation.

### Preferred agents inactivating or stimulating ERG-associated proteins or altering the expression of ERG-associated genes

According to one preferable embodiment, the agent inactivating the HDAC1 protein is an HDAC inhibitor, especially preferably an inhibitor specific for HDAC1. Inhibitors of HDAC classes I (HDACs 1, 2, 3 & 8) and II (HDACs 4, 5, 6, 7, 9 & 10) have recently emerged as potent anti-cancer agents. A proposed mechanism for the anti-tumor effects of HDAC inhibitors is that the accumulation of acetylated histones leads to activation (and repression) of the transcription of a selected number of genes whose expression causes inhibition of tumor cell growth and/or the induction of apoptosis (= programmed cell death). Therefore, HDAC inhibitors have been shown to be potent inducers of growth arrest, differentiation, and/or apoptotic cell death. Some newly synthesized compounds are potentially effective agents for cancer therapy and, possibly, cancer chemoprevention. However, there is only limited information about combination therapy using, for example, HDAC inhibitors and other anti-cancer drugs, or androgen ablation therapy. HDAC inhibitors, both peptides and small molecules are described in the art. HDAC inhibitors currently in clinical trials or pre-clinical investigation include **SAHA** (suberoylanilide hydroxamic acid), **Trichostatin A** (TSA) and it's homologues **CAY10398,** trapoxin A and B; the fatty-acid derivatives Sodium **butyrate,** Sodium **4-phenylbutyrate, Butyrolactone** 3 and **Valproic acid;** or the synthetic benzamide derivatives **MS-275, ITSA1** ((1H-Benzotriazol-1-yl)-2,4-dichlorobenzamide), and **CTPB** (N-(4-Chloro-3-trifluoromethyl-phenyl)-2-ethoxy-6-pentadecyl-benzamide). Furthermore, compounds such as **MC 1293** (3-(4-Toluoyl-1-methyl-1H-2-pyrrolyl)-N-hydroxy-2-propenamid), **Scriptaid, Sirtinol, M344** (4-Dimethylamino-N-(6-hydroxycarbamoylhexyl)-benzamide), **Oxamflatin, Splitomicin, Anacardic acid,** and **Apicidin** are known as potent HDAC inhibitors. Many more compounds may exist that have potent HDAC-Inhibitor properties, but have not been specifically tested. The HDAC inhibitors mentioned above, particularly TSA and MS-275, are also effective to either inactivate or to stimulate other ERG-associated genes than HDAC.

As examples of other agents inactivating the HDAC protein can be mentioned an antibody raised against HDAC, or an aptamer (an oligonucleotide) affecting the protein conformation of HDAC resulting in the inactivation of the same. Several HDAC antibodies are disclosed in the art, and some are commercially available. Also other ERG-associated proteins than HDAC can be inactivated by antibodies and aptamers.

Further, the agent can be an agent down regulating the expression of the HDAC or other ERG-associated gene. Such an agent can, for example, be an antisense oligonucleotide, modified nucleotide, sequence of combination of different kinds of nucleotides to prevent or modify the protein synthesis. The antisense oligonucleotide can be a DNA molecule or an RNA molecule. The agent down regulating the expression of HDAC or other ERG-associated gene can also be a small interfering RNA (siRNA), or a ribozyme complementary to a target region of the mRNA of the protein.

Figure 7 shows the sequences (cDNA and amino acid sequence) of three transcripts of HDAC1. Suitable target regions in the HDAC1mRNA may be found in any of the three transcripts.

In the method described above, the agent inactivating the HDAC1 protein in the patient or the agent down regulating the expression of said protein should preferably be an agent specific for the HDAC1 protein.

The HDAC inhibitors mentioned above are also effective in down regulating the mRNA levels of ERG-associated genes.

The treating method according to this invention can be accomplished either as the sole treating method, or as an adjuvant therapy, combined with other methods such as administration of cytotoxic agents, surgery, radiotherapy, immunotherapy etc.

The therapeutically effective amount of the agent to be given to a patient in need of such treatment may depend upon a number of factors including, for example, the age and weight of the patient, the precise condition requiring treatment and its severity, and the route of administration. The precise amount will ultimately be at the discretion of the attending physician. Thus, practice of the present invention may involve any dose, combination with other therapeutically effective drugs, pharmaceutical formulation or delivery system for parenteral administration. The agent can be administered systemically or locally. As suitable routes of administration can be mentioned oral, intravenous, intramuscular, subcutaneous injection, inhalation, topical, ocular, sublingual, naval, rectal, intraperitoneal delivery and iontophoresis or other transdermal delivery systems.

### Diagnostic methods:

Furthermore, the invention concerns a method for screening of prostate cancer patients with ERG-activation or ERG-translocation in order to evaluate said patients' response to an anti-ERG therapy, optionally in combination with an androgen deprivation therapy, said method being based on use one or more ERG-associated genes and/or one or more ERG-related pathways as a biomarker.
Moreover, the invention concerns methods for assessing the efficacy of a therapy for treating prostate cancer in a patient, assessing progression of prostate cancer in a patient, selecting an agent to be tested for usefulness in the treatment of prostate cancer, and for assessing prostate carcinogenic potential of an agent.

Preferably, the biomarker is an ERG-associated gene that is any of the genes listed in Table 3, or any combination of said genes. Even more preferably, the biomarker is an ERG-associated gene that is any of the genes listed in Table 5, or any combination of said genes.

A particularly preferable biomarker is HDAC1, optionally in combination with one or more of the additional genes listed in Table 3.

The method for monitoring patients can be based on an immunoassay of a sample drawn from the patient, using an antibody raised against an epitope in the protein (for example HDAC1 protein) expressed by the ERG-associated gene.

Alternatively, the method can be based on a hybridising technique or RT-PCR analysis of RNA or DNA of TMPRSS2-ERG fusion or the ERG-associated gene (for example HDAC1) in a sample drawn from the patient.

The method can also be based on the detection of a deregulation of an ERG-related key pathway, particularly one or more of the pathways disclosed in Figure 4. Particularly useful pathways may be WNT, TNF/FAS, apoptosis or HDAC pathway, or a combination thereof.

The invention will be illuminated by the following non-restrictive Experimental Section.

### EXPERIMENTAL SECTION

Translocations fusing the strong androgen-responsive gene *TMPRSS2* with *ERG* or other oncogenic ETS factors may facilitate prostate cancer development. Here, we studied 18 advanced prostate cancers for ETS factor alterations, using RT-PCR and DNA and RNA array technologies, and identified putative ERG downstream gene targets from microarray data of 410 prostate samples. Out of the 27 ETS factors, ERG was most frequently overexpressed. Seven cases showed *TMPRSS2:ERG* gene fusions, whereas the *TMPRSS2:ETV4* fusion was seen in one case. In five out of six tumors with high ERG expression, array-CGH analysis revealed interstitial 2.8 Mb deletions between the TMPRSS2 and ERG loci or smaller, unbalanced rearrangements. *In silico* analysis of the ERG gene co-expression patterns revealed an association with high expression of HDAC1 gene, and low expression of its target genes. Furthermore, we observed increased expression of WNT-associated pathways and downregulation of TNF and cell death pathways. In summary, our data indicate that the *TMPRSS2:ERG* translocation is common in advanced prostate cancer and occurs by virtue of unbalanced genomic rearrangements. Activation of *ERG* via androgen-dependent *TMPRSS2* fusion may lead to epigenetic reprogramming, WNT signaling, and downregulation of cell death pathways, implicating ERG in several hallmarks of cancer with potential therapeutic importance.

### Materials and Methods

### Patient data and prostate cancer cell lines

Nineteen advanced prostate cancer samples were obtained from eighteen patients. The tissue samples included into this analysis were leftovers after the clinical diagnosis was completed; and were used according to the contemporary guidelines and informed consent of the patients was obtained. The frozen tissue blocks were sectioned using a cryostat, and 20 µm sections were collected for DNA and RNA extractions. In addition, five prostate cancer cell lines; VCaP, 22Rv1, DU145, LNCaP and PC-3, were included in the analyses.

### Sample preparation, gene copy number and expression data

DNA was extracted from the samples after overnight proteinase K treatment using standard protocols, whereas total RNA was extracted using either TRIzol (Invitrogen, Carlsbad, CA) or LiCl/urea isolation-based methods. Genome-wide data for DNA copy number and matching gene expression data from the same samples will be described in detail elsewhere^{I}. The data were merged to analyze the corresponding copy number and expression levels for the *ERG, ETV4, ETV1* and *TMPRSS2* loci. Briefly, array-based CGH was performed using Human Genome CGH 44A and 44B oligo microarrays according to the protocol version 2 provided by Agilent Technologies (Agilent Technologies, Palo Alto, CA), with minor modifications. Male genomic DNA (Promega, Madison, WI) was used as reference in all hybridizations. 3 µg of digested tumor DNA and reference DNA were labeled using Cy5-dUTP and Cy3-dUTP incorporation (PerkinElmer, Wellesley, MA) and the Bioprime Array CGH Genomic Labeling Module (Invitrogen, Carlsbad, CA). A laser confocal scanner (Agilent Technologies) was used to obtain signal intensities from targets, and Agilent Feature Extraction software (version 8.1.1.1) was applied using manufacturer's recommended settings. To analyze the aCGH data we used the CGH Analytics software (version 3.2.32, Agilent Technologies).

Gene expression levels were measured using Affymetrix GeneChip Human Genome U133 Plus 2.0 arrays (Affymetrix, Santa Clara, CA). Sample processing and labelling were performed according to the protocol provided by Affymetrix. 3 µg of total RNA from each sample was used for the initial one-cycle cDNA synthesis. Arrays were scanned immediately after staining using a GeneChip scanner (Affymetrix).

### Reverse transcription-polymerase chain reaction (RT-PCR) to identify TMPRSS2-ERG, TMPRSS2-ETV1 and TMPRSS2-ETV4 fusion transcripts

Reverse transcription used 50 ng of total RNA, Sensiscript Reverse transcription kit (Qiagen) and oligo dT-primers. The fusion transcripts were then PCR amplified, using gene specific primers for *TMPRSS2* exon 1 (5'- CAGAGCTGCTAACAGGAGGCGGAGGCGGA-3) (SEQ ID NO. 11), *ERG* cDNA exon 11 (5'-CATAGTAGTAACGGAGGGCGC-3') (SEQ ID NO. 12), *ETV1* cDNA exon 9 (5'-TTGTGGTGGGAAGGGGATGTTT-3') (SEQ ID NO. 13), and *ETV4* cDNA exon 8 (5'-CGAAGTCCGTCTGTTCCTGT-3') (SEQ ID NO. 14). The PCR was performed with Phusion High-Fidelity DNA polymerase (Finnzymes, Espoo, Finland). All PCR experiments included RT-negative controls and a blank with no template. PCR products were isolated from agarose gels, treated with Taq polymerase to generate polyA overhangs, and cloned into pCRII-TOPO cloning vector (Invitrogen, Carlsbad, CA). Sequencing reactions using the same primers as for amplification were prepared by using the ABI BigDye Terminator V3.1 cycle sequencing kit, according to the manufacturer's instructions and analyzed on the ABI 3100 genetic Analyzer (Applied Biosystems).

### Data filtering and normalization, clustering and statistical analysis

Affymetrix U133 Plus 2.0 arrays were normalized using R (8) and the RMA (9) implementation in Bioconductor package affy. Multiple probe sets mapping to the same genes were combined using mean values. Both genes and samples were clustered hierarchically using Euclidean distance and complete linkage analyses.

### In silico analysis of potential ERG target genes

We assembled expression data from of 410 human prostate tissue samples, consisting of 178 normal samples and 232 tumors and metastases (Table 1). We analyzed the patterns of ERG co-expressed genes by four independent methods. First, Significance Analysis of Microarrays (SAM, 10) was performed to identify genes that correlate with ERG in prostate tissues. Prostate samples from HG-U95A platform were divided into ERG positive samples (group 2, n = 16), expressing ERG at higher levels than any of the normal samples, and into ERG negative samples including both normal and tumor tissues (group 1, n= 93). Samples with high level expression of any of the other ETS factors were excluded from the SAM analyses. Another SAM was performed with ERG positive (n=16) vs. negative (n=48) tumor samples only. The false discovery rate was set to zero in both analyses. Second, hierarchial clustering analysis was performed to identify genes co-expressed with ERG in prostate samples in an unsupervised manner. Third, we identified genes whose expression was most closely associated with that of ERG's by using a Perl implementation of Pearson's correlation (correlation factors >0.5 or <-0.5). Finally, Gene Ontology analysis using the DAVID GO analysis tool (http://www.david.niaid.nih.gov) and gene set enrichment analysis (GSEA, Broad Institute of MIT and Harvard) were performed using the same expression data as in the SAM analyses. The data from the three different patient cohorts and the four different analysis methods were overlaid to define the most consistent alterations associated with the ERG gene expression.

**Table 1. Origins of prostate tissue gene expression data used in the in silico studies.**

| **DATA SET** | **SOURCE** | **NORMALS (N)** | **TUMORS AND** **METASTASES (N)** |
|---|---|---|---|
| **1** | 9 published Affymetrix microarray studies (PMID:s 14722351, 11773596, 11904358, 12086878, 12154061, 15075390,15388519, 11742071, 14722351) | 137 | 147 |
| **2** | cDNA microarray study (PMID: 14711987) | 41 | 71 |
| **3** | Advanced prostate cancer samples described in this report (Affymetrix U133 Plus 2.0 array) | 0 | 14 |

### Results and Discussion

### ETS factor gene expression in hormone refractory prostate cancers

Affymetrix gene expression data for 27 ETS family members were determined for the 14 advanced prostate cancer samples with informative CGH profiles (Figure 1). ERG was found to be the most frequently upregulated ETS factor in advanced prostate tumors, including both hormone-refractory (4/9, nos 3, 4, 7 and 13) and untreated clinically advanced prostate cancers (2/5, nos 14 and 16). None of the ETS factors were consistently associated with hormone refractory tumors. Androgen receptor (AR) expression was highly increased in seven of nine hormone refractory prostate cancers (nos 1, 3, 4, 6, 7, 8 and 18; mean value = 1252, S.D. = 794, range 478-2328 vs. nos 2 and 13; mean value = 56, S.D:= 43, range 25-86, Table 2). However, the AR expression levels were not associated with ERG activation. In AR overexpressing cancers, the ERG expression was 102 ± 110 (mean ± S.D) ranging from 16 to 264, whereas in other advanced prostate cancers, ERG expression was 179 ± 208 (range 12-481). Therefore, our results show that the ERG gene overexpression is a frequent event in prostate cancer, but that this alteration is not associated with the hormone-refractory nature of the tumors, nor with the androgen receptor overexpression.

**Table 2. Prostate cancer specimens, array-CGH, expression and RT-PCR results**

| **Sample ID** | **Tumor type**** | **aCGH** **for *AR*** | ***AR*** **expression** | **aCGH** **for ERG region** | ***ERG*** **expression** | ***TMPRSS*** **2:ERGfusion transcript** | ***ETV4*** **expression** | ***TMPR5 S2:ETV*** **4 fusion transcr pt** |
|---|---|---|---|---|---|---|---|---|
| 1 | recurrence, HR | amp | 2168 | - | 23 | - | 30 | - |
| 2 | recurrence, HR | - | 25 | Interstitial deletion between *TMPRSS2* and *ERG* | 12 | - | 22 | - |
| 3 | recurrence, HR | - | 525 | Interstitial deletion between TMPRSS2 and ERG | 140 | + | 29 | - |
| 4 | recurrence, HR | amp | 2328 | Deletions at *ERG* and *TMPRSS2* loci | 236 | + | 22 | - |
| 5 | recurrence, HR | amp | NA | - | NA | - | NA | - |
| 6 | recurrence, HR | amp | 550 | - | 17 | - | 33 | - |
| 7 | recurrence, HR | whole X chr gain | 1623 | - | 264 | + | 25 | - |
| 8 | recurrence, HR | gain | 478 | - | 16 | - | 949 | + |
| 9 | recurrence, HR | NA | NA | NA | NA | - | NA | - |
| 10 | Advanced | - | NA | Interstitial deletion between *TMPRSS2* and *ERG* | NA | + | NA | - |
| 11 | Advanced | - | NA | - | NA | - | NA | - |
| 12a | lung met | - | 159 | - | 15 | - | 41 | - |
| 12b | liver met | - | NA | whole chr 21 loss | NA | - | NA | - |
| 13 | lymph node met, HR | - | 86 | Deletion adjacent to *ERG* | 391 | + | 25 | - |
| 14 | lymph node me | - | 145 | Interstitial deletion between *TMPRSS2* and *ERG* | 481 | + | 21 | - |
| 15 | lymph node me | - | 215 | - | 20 | - | 22 | - |
| 16 | lymph node me | - | 113 | Interstitial deletion between *TMPRSS2* and *ERG* | 317 | + | 18 | - |
| 17 | lymph node me | - | 195 | - | 20 | - | 22 | - |
| 18 | lymph node met, HR | amp | 1093 | Deletion proximal to *ERG* | 16 | - | 117 | - |

| | | | | | | | | |
|---|---|---|---|---|---|---|---|---|
| Abbreviations: amp, amplification; NA, data not available; -, no change | | | | | | | | |

### TMPRSS2-ERG fusion genes in prostate cancer cell lines and tumors.

Nineteen tumor samples and five prostate cancer cell lines were screened for the presence of the three previously identified TMPRSS2-ETS transcription factor fusions by RT-PCR. A fragment of the expected size was amplified with *TMPRSS2:ERG* specific primers from the VCaP cell line (1), whereas the other prostate cancer cell lines analyzed were negative. The *TMPRSS2-ERG* fusion transcript was also detected in seven of the 19 prostate cancer samples (Figure 1, Table 2), indicating that the fusion transcript is expressed in approximately 40 percent of advanced prostate cancers. Six of these samples (nos.3, 4, 7, 13, 14, 16) were strongly positive, whereas in one advanced prostate cancer sample (no. 10), the fusion transcript was expressed at very low levels (data not shown). Sequence analysis of the RT-PCR products indicate that the most common fusion transcript was a fusion of exon 1 of *TMPRSS* with the beginning of exon 4 of *ERG (TMPRSS2:ERGa,* ref 1). In one tumor (no. 10) exon 1 of *TMPRSS2* was fused to the beginning of exon 2 of *ERG* (*TMPRSS2: ERGb,* ref 1). In sample no. 16, the exon 3 of *TMPRSS2 was* fused to the beginning of exon 4 of *ERG.* In tumor no.10, there was also a more complicated fusion consisting of exon 2 of *TMPRSS2* fused to 95 nucleotides identical to a segment of ERG splice form 6 (ERG6 mRNA, AY204740.1 bp:225-286), followed by the entire exon 4 of *ERG.*

### Genomic rearrangements at the ERG locus by array-CGH analysis

Genomic rearrangements, either deletions at the ERG locus or interstitial deletions between the *TMPRSS2* and *ERG* loci, were identified by array CGH in five out of the six samples displaying *TMPRSS2:ERG* gene fusions with high ERG expression (Nos. 3, 4, 13, 14, 16) (Figure 2). This indicates that the ERG activation is not caused by simple balanced translocation, but by a variety of unbalanced genetic rearrangements that bring together these two adjacent loci. The proximity of the two genes (with a genomic distance of only 2.8 Mb), and location in the same DNA strand, may facilitate the fusion gene formation and allow a simple intragenic deletion to activate the *ERG* gene by the fusion to *TMPRSS2.* This was the most prevalent genetic alteration by array-CGH. In one of the tumors (no 4), there were two microdeletions both at the *TMPRSS2* and *ERG* loci (measuring 911 and 159 kb, respectively), suggesting that two unbalanced rearrangements led to fusion gene formation.

### TMPRSS2-ETV1 and ETV4 fusion genes

The occurrence of *TMPRSS2-ETV1* fusion (1, 11) could not be detected in any of our prostate cancer tumor samples. Recently, another rearrangement fusing an 8 kb upstream region of *TMPRSS2* and a third member of the ETS transcription factor family, *ETV4,* was described (2). In two of our tumors (nos. 8 and 18), *ETV4* over-expression was detected. The results from the RT-PCR analyses indicate that tumor 8 contains a *TMPRSS2-ETV4* fusion transcript, but all other samples were negative.

### In silico analysis of ERG co-expressed genes in prostate tumors

The *ERG* gene is the most consistently overexpressed oncogene in malignant epithelial cells of the prostate (4). However, its functional role in prostate cancer development and progression has not yet been clearly determined. To identify potential ERG target genes and deregulated biological processes *in vivo* in uncultured prostate cancers with ERG overexpression, we analyzed gene coexpression data from three different prostate data sets, consisting altogether of 410 prostate tissue samples.

The largest data set (n=284) consisted of nine previously published Affymetrix gene expression studies. These data were normalized to render them directly comparable². Expression data were analyzed by multiple statistical methods to characterize the most consistently ERG associated genes. First, the results from SAM analysis indicated that 136 genes were significantly differentially expressed between ERG positive and negative prostate samples; 92 genes were positively correlating (>1.5 fold change, positive hits), and 44 genes were negatively correlating with ERG (Table 3). SAM was also performed with prostate cancers only (Figure 3). Second, the ERG clusters generated by K-means and hierarchical clustering showed a 46% and 59% percent overlap, respectively, with the positive SAM hit list. Third, ERG correlation analyses across all prostate samples (n=284) or all prostate cancer samples (n=147) were performed. For the top 200 genes correlating with ERG in prostate cancers, there was an 85% overlap with the SAM positive hits using linear correlation and 75% for log-transformed correlation.

For validation, we used an independent large prostate dataset (n=112) based on cDNA microarray analysis (12). Only half of the top 200 positively ERG-correlating genes in the second data set were represented on Affymetrix microarray platforms, and of these 46% overlapped with the positive SAM hits.

The third data set (n=14) consisted of the advanced prostate cancers analyzed in this study. Although the number of samples was small and many samples were derived from hormone refractory prostate cancers, three genes among the top ERG correlating genes were also among the top ten positive SAM hit gene list. The correlation results from the three different datasets overlaid with the SAM results are presented in the Table 3.

**Table 3. The most significant SAM hit genes overlaid with the ERG correlation results obtained from prostate cancers.**

| | | **SAM** | **SAM** | **DATA** **SET 1** | **DATA** **SET 2** | **DATA** **SET 3** |
|---|---|---|---|---|---|---|
| **Gene ID** | **Gene Name** | **Score(d)** | **Fold** **Change** | **Log r** | **Log r** | **Log r** |
| ENSG00000157554 | ERG | 15.35 | 8.10 | P | P | P |
| ENSG00000157911 | PEX10 | 10.77 | 2.21 | P | A | A |
| ENSG00000116478 | HDAC1 | 9.75 | 1.64 | P | P | P |
| ENSG00000104490 | NCALD | 8.41 | 2.25 | P | P | A |
| ENSG00000103769 | RAB11A | 8.18 | 1.74 | P | P | A |
| ENSG00000152270 | PDE3B | 7.84 | 1.97 | P | A | P |
| ENSG00000176871 | WSB2 | 7.36 | 1.96 | P | A | A |
| ENSG00000157388 | CACNA1D | 7.31 | 1.97 | P | P | A |
| ENSG00000146070 | PLA2G7 | 7.21 | 2.80 | P | P | A |
| ENSG00000069122 | GPR116 | 6.99 | 3.21 | P | A | A |
| ENSG00000100105 | ZNF278 | 6.98 | 1.70 | P | A | A |
| ENSG00000073969 | NSF | 6.83 | 1.79 | P | A | A |
| ENSG00000104783 | KCNN4 | 6.65 | 2.52 | P | A | A |
| ENSG00000175130 | MARCKSL1 | 6.59 | 1.58 | P | A | A |
| ENSG00000119888 | TACSTD1 | 6.49 | 2.15 | A | A | A |
| ENSG00000185275 | CD24 | 6.48 | 2.01 | P | A | A |
| ENSG00000106541 | AGR2 | 6.48 | 2.33 | P | A | A |
| ENSG00000164116 | GUCY1A3 | 6.37 | 2.05 | P | A | A |
| ENSG00000096433 | ITPR3 | 6.27 | 1.92 | P | A | A |
| ENSG00000117525 | F3 | 6.19 | 2.33 | P | P | A |
| ENSG00000163501 | IHH | 6.17 | 2.02 | P | A | A |
| ENSG00000196781 | TLE1 | 6.12 | 1.60 | P | P | A |
| ENSG00000084073 | ZMPSTE24 | 6.10 | 1.50 | P | A | A |
| ENSG00000197822 | OCLN | 6.07 | 1.98 | P | P | A |
| ENSG00000134755 | DSC2 | 6.04 | 1.83 | P | A | P |
| ENSG00000169562 | GJB1 | 6.03 | 1.55 | P | A | A |
| ENSG00000148908 | RGS10 | 5.93 | 1.62 | P | A | A |
| ENSG00000198734 | F5 | 5.89 | 2.80 | P | A | A |
| ENSG00000138760 | SCARB2 | 5.88 | 1.67 | A | A | A |
| ENSG00000170745 | KCNS3 | 5.88 | 2.05 | P | P | P |
| ENSG00000139219 | COL2A1 | 5.88 | 1.97 | P | A | A |
| ENSG00000131773 | KHDRBS3 | 5.82 | 2.88 | P | P | A |
| ENSG00000105707 | HPN | 5.77 | 1.78 | P | A | A |
| ENSG00000112091 | HLA-DMB | 5.67 | 1.67 | P | A | A |
| ENSG00000135744 | AGT | 5.65 | 4.58 | A | A | A |
| ENSG00000108091 | CCDC6 | 5.63 | 1.63 | P | A | A |
| ENSG00000196586 | MYO6 | 5.54 | 2.30 | P | P | P |
| ENSG00000171617 | ENC1 | 5.45 | 1.76 | P | A | A |
| ENSG00000170035 | UBE2E3 | 5.44 | 1.85 | P | A | A |
| ENSG00000117143 | UAP1 | 5.38 | 1.72 | P | A | A |
| ENSG00000039068 | CDH1 | 5.37 | 1.54 | A | A | A |
| ENSG00000177425 | PAWR | 5.36 | 1.62 | P | A | A |
| ENSG00000049089 | COL9A2 | 5.29 | 1.80 | P | A | A |
| ENSG00000198648 | STK39 | 5.26 | 1.53 | P | P | A |
| ENSG00000156284 | CLDN8 | 5.19 | 1.82 | P | A | A |
| ENSG00000132437 | DDC | 5.11 | 6.65 | P | A | A |
| ENSG00000123143 | PKN1 | 5.06 | 1.73 | A | A | A |
| ENSG00000163618 | CADPS | 5.04 | 3.65 | P | A | A |
| ENSG00000189058 | APOD | 5.03 | 1.70 | P | P | A |
| ENSG00000111275 | ALDH2 | -6.78 | 0.53 | P | A | A |
| ENSG00000132329 | RAMP1 | -6.73 | 0.47 | P | A | A |
| ENSG00000158747 | NBL1 | -5.95 | 0.64 | P | A | A |
| ENSG00000101951 | PAGE4 | -5.55 | 0.33 | P | A | A |
| ENSG00000145779 | TNFAIP8 | -5.45 | 0.56 | P | P | A |
| ENSG00000185432 | AAM-B | -5.24 | 0.59 | P | A | A |

| | | | | | | |
|---|---|---|---|---|---|---|
| Abbreviations in Table 3: P, present; A, absent | | | | | | |

Histone deacetylase 1 (HDAC1), was the only gene among the top ERG coexpressed genes in all three data sets and therefore was the most consistent feature of ERG overexpressing prostate cancers. By RT-PCR validation of the HDAC1 expression levels, all ERG-positive prostate cancers were strongly HDAC1 positive, whereas ERG-negative tumors showed more variable, but not significantly lower (p=0.0001) expression levels (Figure 5). HDAC1 catalyzes the deacetylation of lysine residues on the N-terminal part of the core histones and other proteins, leading to epigenetic silencing of target genes. HDAC1 has been shown to be strongly expressed in hormone refractory prostate cancers (13). Currently, it remains unclear if HDAC1 is a direct transcriptional target of ERG or whether HDAC1 upregulation results from other changes occurring in ERG overexpressing tumors. ERG has been shown to interact indirectly with HDAC1 via SETDB1 methyl transferase (14, 15).

Results from gene ontology analyses indicated that the "organogenesis" as well as "cell growth and maintenance" were the most significantly (p-values 0.001 and 0.02) overrepresented gene ontology terms in ERG positive tumors. GSEA results, presented in Figure 4, indicated that the WNT and PITX2 pathways were among the most highly enriched pathways in ERG over-expressing tumors (16, 17). The WNT pathway controls organogenesis by inducing e.g. PITX2 transcription factor, which serves as an important modulator of growth control genes (17). HDAC1 itself is linked to these two pathways. The downregulated gene sets in ERG overexpressing tumors included CCR5, cell death, and TNF/FAS. Interestingly, also HDAC pathway with known HDAC target genes and regulators was highlighted by this analysis suggesting that the upregulation of HDAC1 in ERG-positive tumors led, as could be expected, to downregulation of HDAC target genes (18, 19). The genes showing core enrichment in the identified pathways are presented in Table 4.

**Table 4. Genes showing core enrichment in ERG positive prostate cancers identified by the GSEA analysis.**

| Gene set* | Core Enrichment |
|---|---|
| Upregulated | |
| WNT pathway (8/22) | HDAC1, TLE1, AXIN1, CTBPI, CSNK1D, WNT1, CSNK2AI, APC |
| PLCE Pathway (4/11) | ADCY1, PRKACB, GNAS, PTGER1 |
| Mitochondria Pathway (10/19) | BIK, APAF1, BCL2L1, PDCD8, BID, ENDOG, DFFA, DFFB, BCL2, CASP3 |
| MPR Pathway (8/21) | ADCY1, PRKACB, GNAI1, GNB1, CCNB1, RPS6KA1, GNAS, MYT1 |
| PITX2 Pathway (6/15) | HDAC1,AXIN1, TRRAP, WNT1, APC, EP300 |
| Extrinsic Pathway (3/13) | F5, F3, F2R |
| CHREBP Pathway (5/16) | ADCY1, PRKACB, GNB1, GNAS, PRKAA2 |
| Downregulated CCR5 Pathway (9/16) | JUN, PTK2B, CXCR4, CALM3, CCR5, CCL2, CXCL12, SYT1, CCL4 |
| FCER1 Pathway (15/33) | BTK, NFATC1, SOS1, FCER1A, PIK3CA, VAV1, PRKCB1, JUN, PIK3R1, CALM3, LYN, FCER1G, PPP3CC, MAP2K4, SYT1 |
| TNF and FAS network (8/16) | TRAF2, BIRC2, TANK, TRAF5, TRAF1, TNFRSFIA, TNFRSF1B, TNFAIP3 |
| HDAC Pathway (8/26) | MAPK7, PIK3R1, CALM3, PPP3CC, MEF2A, SYT1, IGF1, YWHAH |
| LAIR Pathway (4/14) | SELP, VCAM I, C7, IL6 |
| Cell death (7/13) | EMP1, CLU, FOSL2, CLUL1, RRAGA, OPTN, EMP3 |
| Calcineurin Pathway (8/16) | NFATC1, PRKCA, PRKCB1, SP1, CALM3, PPP3CC, SYT1, CDKN1A |

| | |
|---|---|
| * Numbers in parentheses: number of genes showing core enrichment/total number of genes in the gene set | |

In conclusion, we have taken the first steps to shed new light into the mechanism of disease in ERG-positive prostate cancers by discovering that ERG may contribute to several different phenotypic hallmarks of cancer. Of significant potential therapeutic interest are epigenetic mechanisms, involving HDAC1 upregulation and target gene silencing. HDAC inhibitors have been tested in animal models of prostate cancer and have shown promising anti-tumor activity (20). Consistent with this hypothesis, ERG-positive prostate cancer cell lines VCaP and DuCaP showed strong responses with the anti-HDAC drug Trichostatin A (Figure 6). Based on these early-stage *in vitro* functional studies and the extensive *in vivo* correlations, we suggest that patients with ERG-positive prostate cancer could benefit from epigenetic therapy with HDAC inhibitors and other epigenetic drugs, perhaps in combination with traditional treatments, such as androgen-deprivation therapy. This study also illustrates how the linking a pathogenetic event, the ERG gene activation, with a downstream phenotypic consequence of therapeutic potential, such as the use of epigenetic compounds, could pave the way towards future individualized therapies for human prostate cancer.

### Effect of HDAC-inhibitors, androgen ablation and their combination on VCaP cancer cell proliferation and apoptosis; effect of HDAC-inhibitors or androgen ablation on ERG signature genes

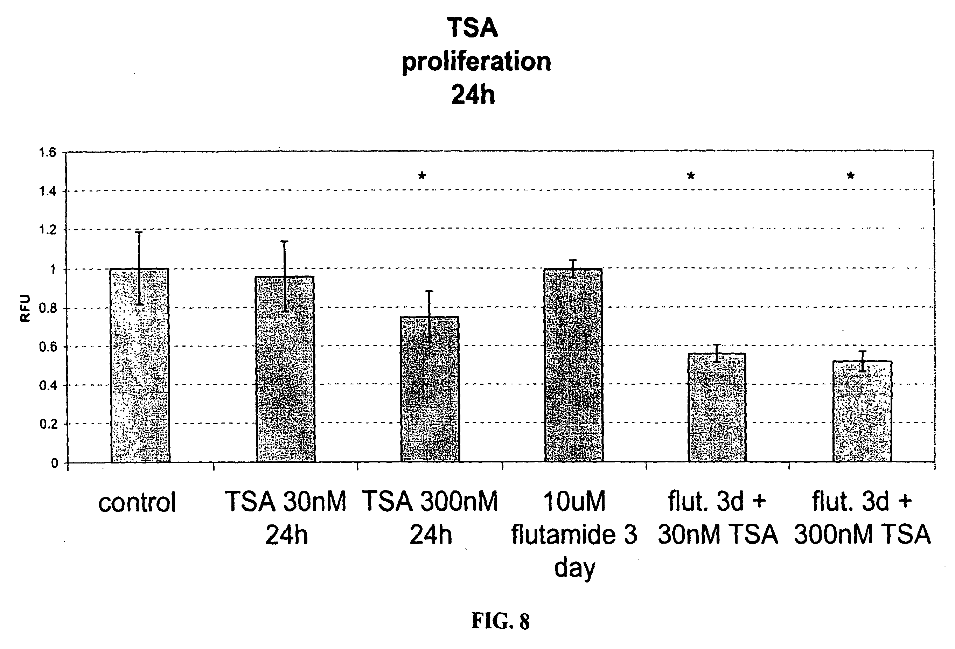

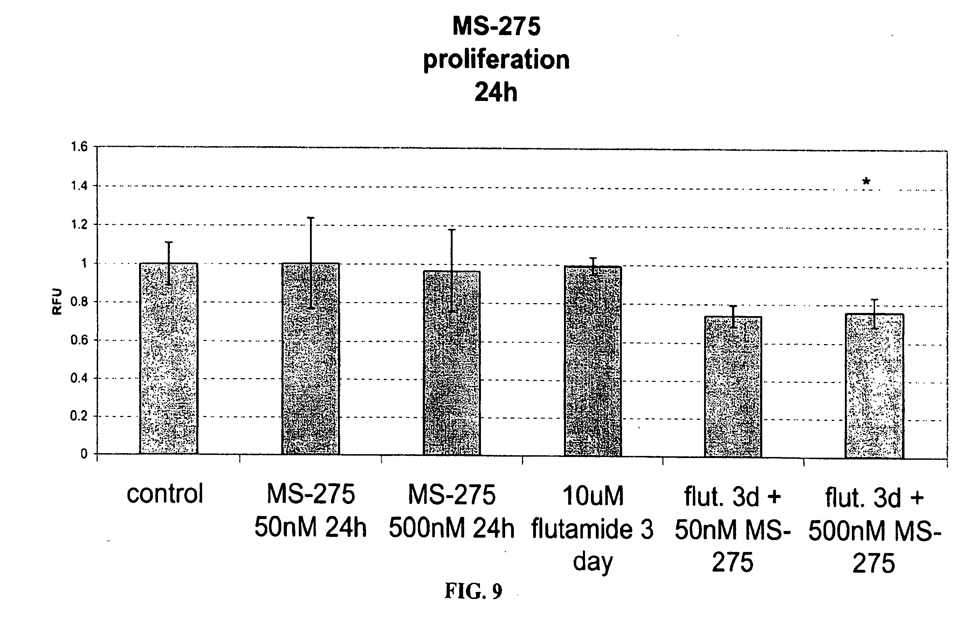

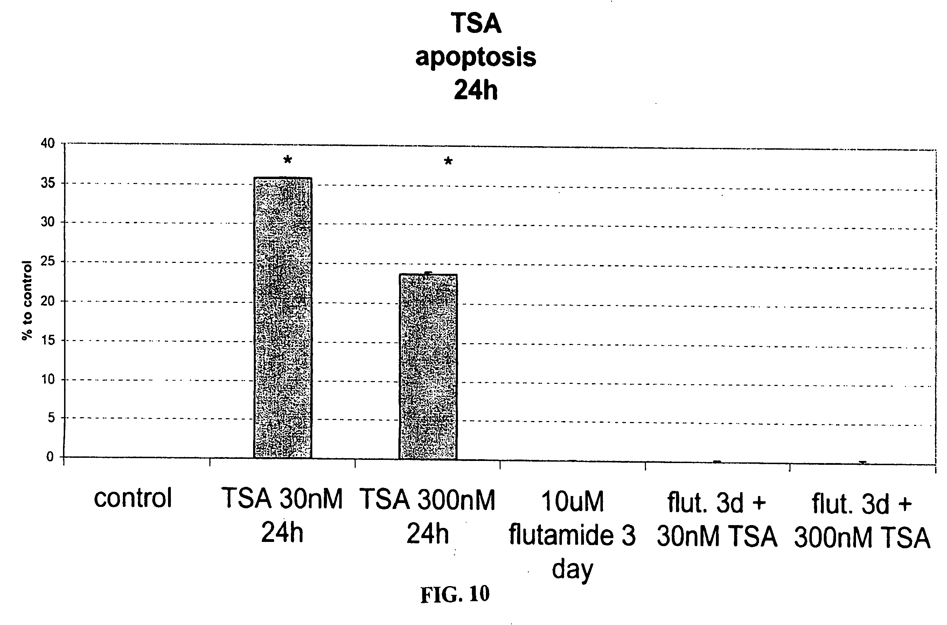

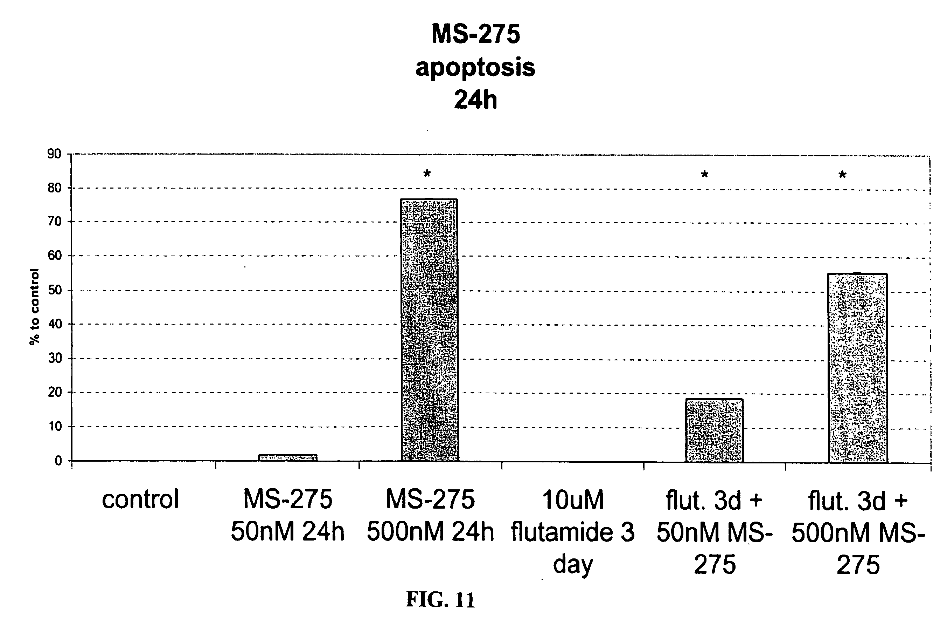

**Cell viability and apoptosis assays.** The combined effects of HDAC inhibitors and androgen-deprivation on cell viability and apoptosis were performed on 384-well plates in which 3000 VCaP cells per well in RPMI 1640 were plated. Cells were left to attach overnight, and medium containing 30 nM, 300 nM and 3 µM trichostatin A (TSA) or 50 nM, 500 nM, and 5 µM MS-275 was added for 24 hours. Hormone- replacement treatment was performed by supplementing the full RPMI 1640 medium with 10 µM of the androgen antagonist flutamide. Cell viability and apoptosis in the response to treatments were measured with homogenous, fluorometric Cell Titer-Blue and Apo-ONE assays (Promega, Madison, WI) according to manufacturer's instructions. As a plate reader, the Envision Multilabel Plate Reader (Perkin-Elmer, Massachusetts, MA) was used. Mean value of at least three independent measurements was used for drawing the plots and Student's T-test was used for calculating the significance of the changes. The effect of the combined HDAC inhibitor and flutamide treatment on cell proliferation is presented in Figures 8 and 9. The raw data was normalized to control and the bars represent standard deviation of the data. The effect of the combined HDAC inhibitor and flutamide treatment on apoptosis is presented in Figures 10 and 11. The data is presented in relation to untreated control as percentages.

**Gene expression analysis by bead-arrays.** Early passage VCaP and LNCaP cells were grown to approximately 70 % confluence before treatments with 300 nM trichostatin A (Sigma) or 500 nM MS-275 (Sigma) for 6 and 12 hours. For androgen ablation, the cells were plated in androgen-deprived medium. 300 nM trichostatin A (Sigma) or 500 nM MS-275 (Sigma) were added for 6 hours and 12 hours before harvesting. Hormone- replacement treatment was performed by supplementing the full RPMI 1640 medium with 10% charcoal-stripped serum. Total RNA was extracted according to the manufacturer's protocol using Trizol reagent (Invitrogen, Carlsbad, CA). Integrity of the RNA prior to hybridization was monitored using a Bioanalyzer 2100 (Agilent, Santa Clara, CA) (according to manufacturer's instruction). 500 ng of purified total RNA was amplified with the TotalPrep Kit (Ambion, Austin, TX) and the biotin labelled cRNA was hybridized to Sentrix HumanRef-8 Expression BeadChips (Illumina, San Diego, CA). The arrays were scanned with the BeadArray Reader (Illumina).

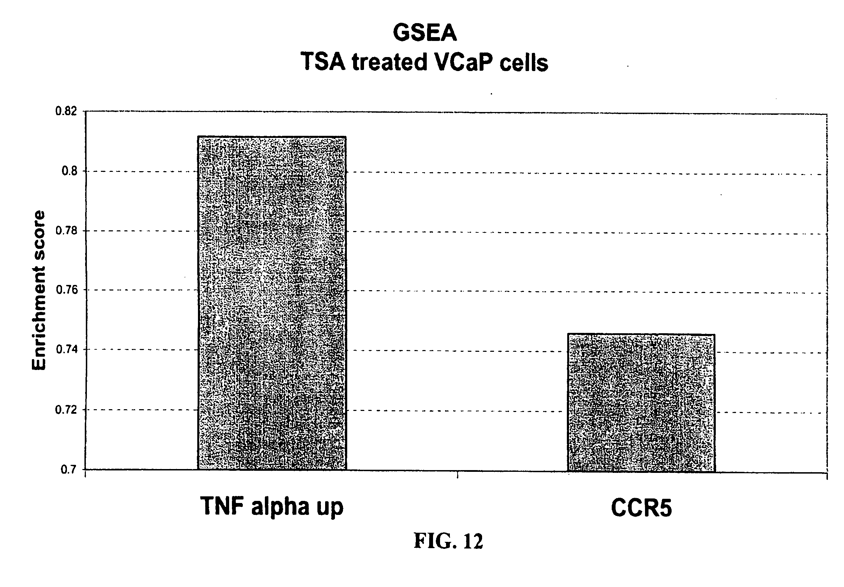

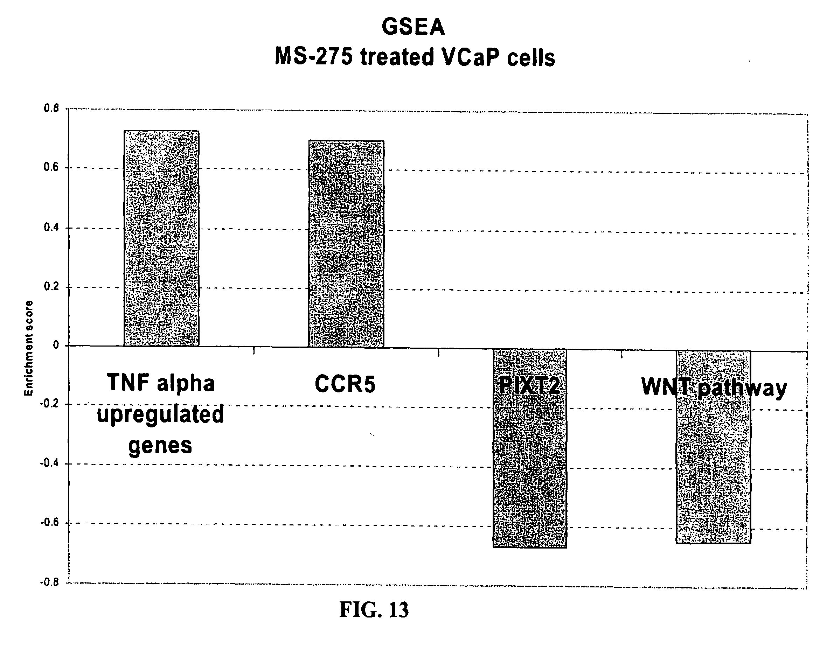

**Statistical analysis.** The raw data was quantile-normalized and analyzed by using the R/Bioconductor software (http://www.r-project.org/, http://www.bioconductor.org/). Mean for ERG signature genes for at least two replicates per condition was calculated and the treatment values were extracted from controls to illustrate the fold changes presented on Table 5. Gene set enrichment analysis (GSEA) was performed for the HDAC inhibitor treatment and androgen ablation data with the GSEA software v2.0.1 (http://www.broad.mit.edu/gsea/). Our own previously defined ERG gene signature was used to evaluate the statistical significance of the signature reversal by HDAC inhibitors and androgen ablation (Table 6.). MSigDB gene sets were used for studying the pathways affected by HDAC inhibitors TSA and MS-275 in ERG-positive prostate cancer cell line VCaP (Figures 12 and 13).

**Table 5. The effect of HDAC inhibitors or androgen ablation to the expression of ERG signature genes in ERG-positive VCaP and ERG-negative LNCaP cells. Values indicate fold changes in comparison to untreated cells (downregulation of ≥1.5 fold shown in grey).**

| | **VCaP** | | | | | **LNCaP** | |
|---|---|---|---|---|---|---|---|
| **Gene** | **Mean fold change of TSA 6h to control** | **Mean fold change of TSA 12h to control** | **Mean fold change of MS-275 6h to control** | **Mean fold change of MS-275 12h to control** | **Mean fold change of 72 h androgen ablation to control** | **Mean fold change of TSA 12h to control** | **Mean fold change of MS-275 6h to control** |
| **ERG** | -1.9 | -2.4 | -1.8 | -0.8 | -2.1 | 1.1 | 1.0 |
| **PEX10** | -1.9 | -1.7 | -1.2 | -0.2 | -1.3 | 1.0 | -1.7 |
| **HDAC1** | -1.3 | -1.0 | -1.2 | -0.2 | -1.4 | 1.1 | 1.0 |
| **NCALD** | -2.9 | -3.8 | 1.2 | 2.2 | -1.0 | 1.0 | 1.7 |
| **RAB11A** | -1.4 | -1.5 | -1.3 | -0.3 | -1.3 | 1.1 | -1.6 |
| **PDE3B** | -1.0 | -1.6 | 1.5 | 2.5 | -1.4 | 1.1 | 1.1 |
| **WSB2** | -1.8 | -2.7 | -1.3 | -0.3 | -1.4 | -1.1 | -1.5 |
| **CACNA1D** | 1.2 | -1.1 | 1.4 | 2.4 | 1.0 | 1.0 | 1.0 |
| **PLA2G7** | -2.6 | -5.6 | 1.4 | 2.4 | 1.1 | 1.3 | 2.4 |
| **GPR116** | -1.3 | -1.4 | 2.4 | 3.4 | 1.3 | -1.0 | 1.0 |
| **ZNF278** | -1.5 | -1.5 | -1.1 | -0.1 | -1.2 | 1.3 | -1.4 |
| **NSF** | -1.0 | 1.2 | 1.2 | 2.2 | 1.1 | -1.1 | 1.1 |
| **KCNN4** | 1.0 | -1.1 | -1.0 | 0.0 | 1.1 | -1.1 | -1.1 |
| **TACSTD1** | -1.1 | -1.7 | 1.1 | 2.1 | 1.0 | 1.0 | 1.3 |
| **CD24** | 1.5 | -1.3 | 2.7 | 3.7 | 2.4 | 2.1 | 7.6 |
| **AGR2** | 1.3 | 1.0 | 3.8 | 4.8 | 2.0 | 1.2 | 2.3 |
| **GUCY1A3** | -4.9 | -5.5 | 1.6 | 2.6 | 2.2 | 2.0 | -1.2 |
| **ITPR3** | -1.4 | -1.9 | -1.2 | -0.2 | -1.3 | -1.0 | -1.0 |
| **F3** | 1.0 | -1.2 | 2.5 | 3.5 | 2.0 | -1.0 | 1.3 |
| **IHH** | -2.3 | -2.6 | -2.2 | -1.2 | -1.1 | -1.0 | 1.0 |
| **TLE1** | 1.3 | 1.4 | 6.7 | 7.7 | 3.9 | 0.0 | 0.0 |
| **ZMPSTE24** | -1.2 | -1.2 | -1.1 | -0.1 | -1.4 | -1.0 | -1.4 |
| **OCLN** | -1.5 | -1.7 | -1.1 | -0.1 | 1.0 | 0.0 | 0.0 |
| **DSC2** | -1.9 | -3.0 | 1.6 | 2.6 | 1.1 | 1.5 | -1.2 |
| **GJB1** | -2.5 | -3.0 | -1.5 | -0.5 | -1.8 | -1.0 | -1.2 |
| **RGS10** | 1.3 | 1.0 | 1.3 | 2.3 | 1.3 | 1.1 | 1.3 |
| **F5** | -1.0 | -1.4 | 3.0 | 4.0 | 1.6 | 1.0 | 1.6 |
| **SCARB2** | -1.3 | -1.6 | -1.0 | 0.0 | 1.0 | 1.0 | 1.0 |
| **KCNS3** | -2.0 | -1.8 | -1.2 | -0.2 | -1.2 | -1.2 | 2.2 |
| **COL2A1** | 1.1 | 1.0 | 1.7 | 2.7 | 1.5 | -1.1 | -1.0 |
| **KHDRBS3** | 2.3 | 1.6 | 4.7 | 5.7 | 3.5 | 1.3 | 3.2 |
| **HPN** | -1.3 | -1.4 | 1.1 | 2.1 | 1.2 | -1.2 | -1.3 |
| **HLA-DMB** | -1.7 | -1.6 | -1.2 | -0.2 | -2.9 | 1.2 | 2.1 |
| **AGT** | 1.0 | -1.1 | 1.3 | 2.3 | 1.2 | 1.2 | 2.3 |
| **CCDC6** | 1.4 | 1.2 | 1.3 | 2.3 | 1.3 | 1.2 | 1.3 |
| **MYO6** | -1.3 | -1.7 | 1.1 | 2.1 | -1.0 | 1.2 | -1.1 |
| **ENC1** | -2.5 | -2.5 | 2.1 | 3.1 | 1.3 | 1.9 | 3.6 |
| **UBE2E3** | -1.3 | -1.4 | 1.3 | 2.3 | 1.2 | 1.1 | -1.0 |
| **UAP1** | 1.3 | 1.3 | 1.2 | 2.2 | -1.0 | 1.1 | 1.0 |
| **CDH1** | 1.3 | 1.4 | 1.2 | 2.2 | 1.2 | 1.1 | 1.1 |
| **PAWR** | -1.1 | -1.2 | 1.4 | 2.4 | 1.1 | 1.1 | 2.1 |
| **COL9A2** | -1.3 | -1.3 | 2.0 | 3.0 | 1.8 | 1.2 | -1.0 |
| **STK39** | -1.6 | -1.9 | 1.3 | 2.3 | 1.1 | 1.7 | 1.2 |
| **CLDN8** | 1.2 | 1.1 | 1.7 | 2.7 | 1.3 | 1.3 | 1.9 |
| **DDC** | -1.7 | -1.9 | 1.6 | 2.6 | 3.3 | -1.0 | -1.1 |
| **CADPS** | -1.4 | -1.4 | -1.1 | -0.1 | 1.0 | 1.0 | -1.0 |
| **APOD** | -1.5 | -1.3 | -1.0 | 0.0 | 1.1 | 1.1 | 1.8 |
| **ALDH2** | 2.3 | 2.3 | 1.9 | 2.9 | 1.7 | 1.0 | 1.1 |
| **RAMP1** | -1.8 | -3.1 | -1.1 | -0.1 | 1.1 | -1.2 | -1.2 |
| **NBL1** | 1.1 | -1.0 | 1.3 | 2.3 | 1.7 | 1.0 | -1.1 |
| **TNFAIP8** | 1.3 | 1.5 | 1.8 | 2.8 | 1.5 | -1.3 | 1.1 |

**Table 6. Statistical significance of the ERG signature reversal and the number of genes affected identified by the GSEA analysis.**

| **Treatment** | **Gene set*** | **ES** | **NES** | **NOM p-val** | **FDR q-val** |
|---|---|---|---|---|---|
| **TSA 12h** | 29/47 | -0.75 | -1.63 | 0 | 0 |
| **MS-27512h** | 21/47 | 0.55 | 1.37 | 0.02 | 0.04 |
| **72 h androgen ablation** | 18/47 | -0.38 | -1.20 | 0.14 | 0.14 |

| | | | | | |
|---|---|---|---|---|---|
| * Number of genes showing core enrichment/total number of genes in the ERG gene set Enrichement score (ES) Normalized enrichment score (NES) Nominal p-value (NOM p-val) False discovery rate (FDR) | | | | | |

It will be appreciated that the methods of the present invention can be incorporated in the form of a variety of embodiments, only a few of which are disclosed herein. It will be apparent for the expert skilled in the field that other embodiments exist and do not depart from the spirit of the invention. Thus, the described embodiments are illustrative and should not be construed as restrictive.

**Table 7. Explanation of gene names, genomic database IDs, and abbreviations**

| **ENSEMBL ID** | **Symbol (HUGO)** | **NCBI UniGene Cluster ID** | **Name (HUGO approved)** | **ENTREZ** |
|---|---|---|---|---|
| ENSG00000157554 | ERG | Hs.473819 | V-ets erythroblastosis virus E26 oncogene like | 2078 |
| ENSG00000157911 | PEX10 | Hs.591454 | Peroxisome biogenesis factor 10 | 5192 |
| ENSG00000116478 | HDAC1 | Hs.88556 | Histone deacetylase 1 | 3065 |
| ENSG00000104490 | NCALD | Hs.492427 | Neurocalcin delta | 83988 |
| ENSG00000103769 | RAB11A | Hs.321541 | RAB11A, member RAS oncogene family | 8766 |
| ENSG00000152270 | PDE3B | Hs.445711 | Phosphodiesterase 3B, cGMP-inhibited | 5140 |
| ENSG00000176871 | WSB2 | Hs.506985 | WD repeat and SOCS box-containing 2 | 55884 |
| ENSG00000157388 | CACNA1D | Hs.476358 | Calcium channel, voltage-dependent, L type, alpha 1 D subunit | 776 |
| ENSG00000146070 | PLA2G7 | Hs.584823 | Phospholipase A2, group VII | 7941 |
| ENSG00000069122 | GPR116 | Hs.362806 | G protein-coupled receptor 116 | 221395 |
| ENSG00000100105 | ZNF278 | Hs.517557 | Zinc finger protein 278 | 23598 |
| ENSG00000073969 | NSF | Hs.431279 | N-ethylmaleimide-sensitive factor | 4905 |
| ENSG00000104783 | KCNN4 | Hs.10082 | Potassium interm.e/small conductance calcium-activated channel, subfamily N, member 4 | 3783 |
| ENSG00000175130 | MARCKSL1 | Hs.75061 | MARCKS-like 1 | 65108 |
| ENSG00000119888 | TACSTD1 | Hs.542050 | Tumor-associated calcium signal transducer 1 | 4072 |
| ENSG00000185275 | CD24 | Hs.375108 | CD24 molecule | 934 |
| ENSG00000106541 | AGR2 | Hs.530009 | Anterior gradient 2 homolog (Xenopus laevis) | 10551 |
| ENSG00000164116 | GUCY1A3 | Hs.24258 | Guanylate cyclase 1, soluble, alpha 3 | 2982 |
| ENSG00000096433 | ITPR3 | Hs.65758 | Inositol 1,4,5-triphosphate receptor, type 3 | 3710 |
| ENSG00000117525 | F3 | Hs.62192 | Coagulation factor III (thromboplastin, tissue factor) | 2152 |
| ENSG00000163501 | IHH | Hs.369782 | Indian hedgehog homolog (Drosophila) | 3549 |
| ENSG00000196781 | TLE1 | Hs.197320 | Transducin-like enhancer of split 1 (E(sp1) homolog, Drosophila) | 7088 |
| ENSG00000084073 | ZMPSTE24 | | CAAX prenyl protease 1 homolog | |
| ENSG00000197822 | OCLN | Hs.592605 | Occludin | 4950 |
| ENSG00000134755 | DSC2 | Hs.95612 | Desmocollin 2 | 1824 |
| ENSG00000169562 | GJB1 | Hs.333303 | Gap junction protein, beta 1, 32kDa (connexin 32, Charcot-Marie-Tooth neuropathy | 2705 |
| ENSG00000148908 | RGS10 | Hs.501200 | Regulator of G-protein signalling 10 | 6001 |
| ENSG00000198734 | F5 | Hs.30054 | Coagulation factor V (proaccelerin, labile factor) | 2153 |
| ENSG00000138760 | SCARB2 | | Lysosome membrane protein II | |
| ENSG00000170745 | KCNS3 | Hs.414489 | Potassium voltage-gated channel, delayed-rectifier, subfamily S, member 3 | 3790 |
| ENSG00000139219 | COL2A1 | Hs.408182 | Collagen, type II, alpha 1 (primary osteoarthritis, spondyloepiphyseal dysplasia, congenital) | 1280 |
| ENSG00000131773 | KHDRBS3 | Hs.444558 | KH domain containing, RNA binding, signal transduction associated 3 | 10656 |
| ENSG00000105707 | HPN | Hs.182385 | Hepsin (transmembrane protease, serine 1) | 3249 |
| ENSG00000112091 | HLA-DMB | Hs.351279 | Major histocompatibility complex, class II, DM beta | 3109 |
| ENSG00000135744 | AGT | Hs.19383 | Angiotensinogen (serpin peptidase inhibitor, clade A, member 8) | 183 |
| ENSG00000108091 | CCDC6 | Hs.591360 | Coiled-coil domain containing 6 | 8030 |
| ENSG00000196586 | MYO6 | Hs.149387 | Myosin VI | 4646 |
| ENSG00000171617 | ENC1 | Hs.104925 | Ectodermal-neural cortex (with BTB-like domain) | 8507 |
| ENSG00000170035 | UBE2E3 | Hs.470804 | Ubiquitin-conjugating enzyme E2E 3 (UBC4/5 homolog, yeast) | 10477 |
| ENSG00000117143 | UAP1 | Hs.492859 | UDP-N-acteylglucosamine pyrophosphorylase 1 | 6675 |
| ENSG00000039068 | CDH1 | Hs.461086 | Cadherin 1, type 1, E-cadherin (epithelial) | 999 |
| ENSG00000177425 | PAWR | Hs.406074 | PRKC, apoptosis, WT1, regulator | 5074 |
| ENSG00000049089 | COL9A2 | Hs.418012 | Collagen, type IX, alpha 2 | 1298 |
| ENSG00000198648 | STK39 | Hs.276271 | Serine threonine kinase 39 (STE20/SPS1 homolog, yeast) | 27347 |
| ENSG00000156284 | CLDN8 | Hs.162209 | Claudin 8 | 9073 |
| ENSG00000132437 | DDC | Hs.359698 | Dopa decarboxylase (aromatic L-amino acid decarboxylase) | 1644 |
| ENSG00000123143 | PKN1 | Hs.466044 | Protein kinase N1 | 5585 |
| ENSG00000163618 | CADPS | Hs.127013 | Ca2+-dependent secretion activator | 8618 |
| ENSG00000189058 | APOD | Hs.522555 | Apolipoprotein D | 347 |
| ENSG00000111275 | ALDH2 | Hs.632733 | Aldehyde dehydrogenase 2 family (mitochondrial) | 217 |
| ENSG00000132329 | RAMP1 | Hs.471783 | Receptor (calcitonin) activity modifying protein 1 | 10267 |
| ENSG00000158747 | NBL1 | Hs.632384 | Neuroblastoma, suppression of tumorigenicity 1 | 4681 |
| ENSG00000101951 | PAGE4 | Hs.441038 | P antigen family, member 4 (prostate associated) | 9506 |
| ENSG00000145779 | TNFAIP8 | Hs.271955 | Tumor necrosis factor, alpha-induced protein 8 | 25816 |
| ENSG00000185432 | AAM-B | | Methyltransferase like 7A, UbiE1 | |

### REFERENCES

**1.** Tomlins SA, Rhodes DR, Perner S, et al. Recurrent fusion of TMPRSS2 and ETS transcription factor genes in prostate cancer. Science 2005;310:644-8.
**2.** Tomlins SA, Mehra R, Rhodes DR, et al. TMPRSS2:ETV4 gene fusions define a third molecular subtype of prostate cancer. Cancer Res 2006;66:3396-400.
**3.** Vaarala MH, Porvari K, Kyllönen A, Lukkarinen O, Vihko O. The TMPRSS2 gene encoding transmembrane serine protease is overexpressed in a majority of prostate cancer patients: detection of mutated TMPRSS2 form in a case of aggressive disease. Int J Cancer 2001;94:705-10.
**4.** Petrovics G, Liu A, Shaheduzzaman S, et al. Frequent overexpression of ETS-related gene-1 (ERG1) in prostate cancer transcriptome. Oncogene 2005;24:3847-52.
**5.** Oikawa T, Yamada T. Molecular biology of the Ets family of transcription factors. Gene 2003;303:11-34.
**6.** Yagasaki F, Wakao D, Yokoyama Y, et al. Fusion of ETV6 to fibroblast growth factor receptor 3 in peripheral T-cell lymphoma with a t(4;12)(p16;p13) chromosomal translocation. Cancer Res 2001;61:8371-4.
**7.** Tognon C, Knezevich SR, Huntsman D, et al. Expression of the ETV6-NTRK3 gene fusion as a primary event in human secretory breast carcinoma. Cancer Cell 2002;2:367-76.
**8.** R Development Core Team: R: a language and.environment for statistical computing. Vienna, Austria: R Foundation for Statistical Computing 2006.
**9.** Irizarry RA, Bolstad BM, Collin F, Cope LM, Hobbs B, Speed TP. Summaries of Affymetrix GeneChip probe level data. Nucleic Acids Res 2003;31:el5.
**10.** Tusher VG, Tibshirani R, Chu G. Significance analysis of microarrays applied to the ionizing radiation response. Proc Natl Acad Sci USA 2001;98:5116-21.
**11.** Soller MJ, Isaksson M, Elfving P, Soller W, Lundgren R, Panagopoulos I. Confirmation of the high frequency of the TMPRSS2/ERG fusion gene in prostate cancer. Genes Chrom Cancer 2006;45:717-9.
**12.** Lapointe J, Li C, Higgins JP, et al. Gene expression profiling identifies clinically relevant subtypes of prostate cancer. Proc Natl Acad Sci USA. 2004; 101:811-6.
**13.** Halkidou K, Gaughan L, Cook S, Leung HY, Neal DE, Robson CN.. Upregulation and nuclear recruitment of HDAC1 in hormone refractory prostate cancer. The prostate 2004;59:177-89.
**14.** Yang L, Xia L, Wu DY,et al. Molecular cloning of ESET, a novel histone H3-specific methyltransferase that interacts with ERG transcription factor. Oncogene. 2002;21:148-52.
**15.** Yang L, Mei Q, Zielinska-Kwiatkowska A, et al. An ERG (ets-related gene)-associated histone methyltransferase interacts with histone deacetylases 1/2 and transcription co-repressors-mSin3A/B. Biochem J. 2003;369:651-7.
**16.** Sierra J, Yoshida T, Joazeiro CA, Jones KA. The APC tumor suppressor counteracts beta-catenin activation and H3K4 methylation at Wnt target genes. Genes Dev 2006;20:586-600.
**17.** Kioussi C, Briata P, Baek SH, et al. Identification of a Wnt/Dvi/beta-Catenin --> Pitx2 pathway mediating cell-type-specific proliferation during development. Cell 2002;111:673-85.
**18.** Sparrow DB, Miska EA, Langley E, et al. MEF-2 function is modified by a novel co-repressor, MITR. EMBO J. 1999;18:5085-98.
**19.** Ota H, Tokunaga E, Chang K, et al. Sirt1 inhibitor, Sirtinol, induces senescence-like growth arrest with attenuated Ras-MAPK signaling in human cancer cells. Oncogene. 2006;25:176-85.
**20.** Li LC, Carroll PR, Dahiya R. Epigenetic changes in prostate cancer: implication for diagnosis and treatment. J Natl Cancer Inst. 2005;97:103-15.

## Claims

1. An *in vitro* method for screening of prostate cancer patients for ERG-activation or ERG-translocation in order to evaluate said patients' response to an anti-ERG therapy, optionally in combination with an androgen deprivation therapy, said method being based on use of one or more ERG-associated genes and optionally additionally one or more ERG-related pathways as a biomarker, wherein at least one ERG-associated gene HDAC1 is used.

2. The method according to claim 1 wherein more than one ERG-associated gene is used and the additional ERG-associated gene is any of the genes additional to HDAC1 listed in Table 3, or any combination of said genes, preferably any of the genes additional to HDAC1 listed in Table 5, or any combination of said genes.

3. The method according to claim 2, wherein said method is based on an immunoassay of a sample drawn from the patient, using an antibody raised against an epitope in the HDAC1 protein.

4. The method according to claim 2, wherein said method is based on hybridising technique or RT-PCR analysis of RNA or DNA of HDAC1 in a sample drawn from the patient.

5. The method according to any of claims 2 to 4 wherein the additional ERG-associated gene or genes are selected from the group consisting of NCALD, PDE3B, PEX10, PLA2G7, RAB11A, HLA-DMB, ALDH2, CACNA1D, CADPS, GPR116 and any combination thereof.

6. The method according to claim 1 wherein said method is based on the detection of a deregulation of an ERG-related key pathway.

7. The method according to claim 6 wherein the key pathway is one or more of the pathways disclosed in Figure 4.

8. The method according to claim 7 wherein the pathway is WNT, TNF/FAS, apoptosis or HDAC pathway, or a combination thereof.

9. An *in vitro* method for assessing the efficacy of a therapy for treating prostate cancer in a patient with ERG-activation or ERG-translocation, said method comprising comparing expression of at least one biomarker, which is an ERG-associated gene and optionally additionally an ERG-related pathway, in a first sample obtained from the patient prior to providing at least a portion of said therapy to the patient, and the expression of said biomarker or biomarkers in a second sample obtained from the patient at a later stage of said therapy, wherein at least one ERG-associated gene HDAC1 is one biomarker.

10. An *in vitro* method for assessing progression of prostate cancer in a patient with ERG-activation or ERG-translocation, comprising the steps of: a) detecting in a sample from the patient at a first time point, the expression of a biomarker HDAC1, which is an ERG-associated gene,
b) repeating the detection of expression of said biomarker HDAC1 at a subsequent time point in time, and
c) comparing the level of expression detected in the first and second detection steps, thereby monitoring the progression of prostate cancer in the patient.

11. An *in vitro* method for selecting an agent to be tested for usefulness in the treatment of prostate cancer in patients with ERG-activation or ERG-translocation, said method comprising the steps of
a) dividing a sample, drawn from the patient and/or comprising prostate cancer cells, in aliquots,
b) separately maintaining all sample aliquots in the presence of different test agents,
c) comparing the expression of at least one biomarker, which is an ERG-associated gene and/or an ERG-related pathway, in each of the aliquots, and
d) selecting as agent one that reverses the expression of said biomarker,
wherein HDAC1, an ERG-associated gene, is one biomarker.

12. An *in vitro* method for assessing the prostate carcinogenic potential of an agent in patients with ERG-activation or ERG-translocation, said method comprising the steps of
a) maintaining separate aliquots of prostate cells in the presence or absence of an agent, the carcinogenic potential of which is to be tested, and
b) comparing expression of a biomarker HDAC1, which is an ERG-associated gene and optionally additionally an ERG-related pathway, in each of the aliquots, and
c) using an altered level of expression of said biomarker HDAC1 maintained in the presence of said agent, relative to that of the aliquot maintained in the absence of said agent, is an indication that the agent possesses prostate carcinogenic potential.

13. Use of an agent i) inactivating, stimulating or altering the expression of an ERG-associated gene or genes listed in Table 3, or protein in a prostate cancer patient, and optionally additionally ii) manipulating an ERG-related pathway in said patient; optionally in combination with an agent reducing the androgen level, wherein at least the HDAC1 gene is inactivate, stimulated or altered, for the manufacture of a pharmaceutical composition useful in a method for treatment of prostate cancer in a patient with confirmed ERG-activation or ERG-translocation.

14. The use according to claim 13 wherein said patient is a carrier of the TMPRSS2-ERG fusion gene.

15. The use according to claim 13 wherein an ERG-associated gene, other than the HDAC1 gene, is any of the genes, other than the ERG and HDAC1 genes, listed in Table 3, or any combination of said genes, preferably any of the genes listed in Table 5, or any combination of said genes.

16. The use according to claim 13, comprising additionally administering an effective amount of an agent reducing the androgen level in said patient.

17. The use according to claim 18, comprising additionally administering an effective amount of an agent reducing the androgen level in said patient.

18. The use according to claim 16, wherein the agent inactivating the HDAC1 protein is selected from the group consisting of a peptide, a small molecule, an antibody or an aptamer.

19. The use according to claim 13, wherein the expression of the HDAC1 protein is down regulated and the agent down regulating the expression of the HDAC1 protein is an antisense oligonucleotide, a small interfering RNA (siRNA), or a ribozyme complementary to a target region of the mRNA of said protein.

20. The use according to claim 13, wherein the agent is specific for the HDAC1 protein.
